(19) Europäisches Patentamt
European Patent Office
Office européen des brevets

(11) EP 2 827 188 A1

(12) **EUROPEAN PATENT APPLICATION**
published in accordance with Art. 153(4) EPC

(43) Date of publication:
**21.01.2015 Bulletin 2015/04**

(21) Application number: **13760369.2**

(22) Date of filing: **13.03.2013**

(51) Int Cl.:
*G02F 1/1337* (2006.01) *C08G 73/10* (2006.01)

(86) International application number:
**PCT/JP2013/057039**

(87) International publication number:
**WO 2013/137333 (19.09.2013 Gazette 2013/38)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **16.03.2012 JP 2012061073**

(71) Applicant: **Soki Corporation**
**Machida-shi, Tokyo 194-0043 (JP)**

(72) Inventors:
- **HOSAKA, Yoshihiro**
  **Machida-shi**
  **Tokyo 194-0043 (JP)**
- **SATO, Michihiko**
  **Shiki-shi**
  **Saitama 353-0007 (JP)**
- **WU Mo**
  **Shanghai 200031 (CN)**

(74) Representative: **Winter, Brandl, Fürniss, Hübner, Röss, Kaiser, Polte - Partnerschaft mbB**
**Patent- und Rechtsanwaltskanzlei**
**Alois-Steinecker-Strasse 22**
**85354 Freising (DE)**

(54) **COMPOSITION FOR FORMING LIQUID CRYSTAL ALIGNMENT FILM AND LIQUID CRYSTAL DISPLAY ELEMENT**

(57) The present invention, which enables to align a liquid crystal molecule, when no voltage is applied thereto, and also to control pretilt angle of the liquid crystal molecule, relates to a composition for a liquid crystal alignment film comprising components represented by the following (A) to (D):
(A) a meta-phenylene diamine derivative represented by the general formula [1];
(B) a meta-phenylene diamine derivative represented by the general formula [2];
(C) a para-arylene diamine represented by the general formula [3];
(D) a tetracarboxylic acid represented by the general formula [4] or a tetracarboxylic acid anhydride represented by the general formula [4'];

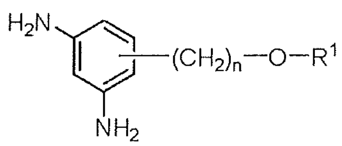 [1] 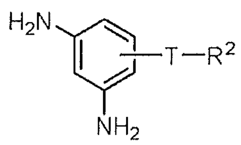 [2]

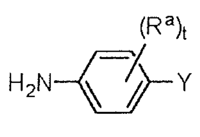 [3] 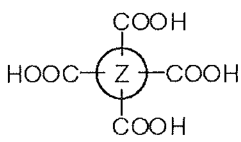 [4] 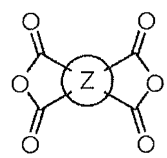 [4']

(wherein $R^1$ represents an alkyl group having 1 to 6 carbon atoms or the like, $R^2$ represents an alkyl group having 8 to 20 carbon atoms or the like, t moieties of $R^a$ represent an alkyl group having 1 to 3 carbon atoms or the like, n represents

EP 2 827 188 A1

an integer of 1 to 3, t represents an integer of 0 to 4, T represents an oxygen atom or the like, Y represents an amino group, and Z represents a tetravalent hydrocarbon group.).

## Description

### TECHNICAL FIELD

The present invention relates to a composition for a liquid crystal alignment film to be used in manufacturing a liquid crystal panel, a polyamic acid or a polyimide obtained from the composition, a liquid crystal aligning agent comprising the polyamic acid or the polyimide, a liquid crystal alignment film prepared from the liquid crystal aligning agent, a method for producing the liquid crystal alignment film, and a liquid crystal display element characterized by being provided with the liquid crystal alignment film. In more detail, the present invention relates to a composition and the like for a liquid crystal alignment film, which enables to control pretilt angle of a liquid crystal molecule at predetermined angle, when no voltage is applied thereto.

### BACKGROUND ART

A liquid crystal display has become an essential display in a monitor for a desktop type personal computer or a notebook-size personal computer, a digital camera, car navigation or the like, and in recent years, the liquid crystal display has also become common in TV.

In liquid crystal display, various display modes have been adopted, depending on the performance required such as contrast, viewing angle, response speed. Specific examples of the display modes include, for example, 1) a TN (Twisted Nematic) mode using a liquid crystal molecule where liquid crystal alignment in no application of voltage is twisted by 90 degrees in a horizontal direction between substrates which sandwich the liquid crystal, 2) an STN (Super Twisted Nematic) mode using a liquid crystal molecule where liquid crystal alignment in no application of voltage is twisted by 180 to 270 degrees in a horizontal direction between substrates which sandwich the liquid crystal, 3) an IPS (In-Plane Switching) mode where a liquid crystal molecule is aligned in a horizontal direction in the plane by applying electric field in a plane direction of substrates which sandwich the liquid crystal molecule, 4) a VA (Vertically Aligned) mode using a liquid crystal molecule aligning vertically relative to a substrate in no application of voltage, and the like, and difference of each mode is based on difference how to move the alignment of the liquid crystal molecule (for example, NON PATENT LITERATURE 1 or the like).

In the TN mode and the STN mode, white display is provided in no application of voltage, black display is provided in application of voltage. In a liquid crystal of such the TN mode and the STN mode, because the liquid crystal molecule does not become completely vertical relative to a substrate even in a state where the voltage is applied to display a black state, visual performance of the liquid crystal molecule shall vary depending on the angle viewing a screen. That is, there is great dependency of viewing angle due to generation of difference in light quantity transmitting a liquid crystal cell (viewing angle is narrow).

In contrast, in the IPS mode, because the liquid crystal molecule rotates in a horizontal direction relative to a substrate by application / no application of voltage, dependency of viewing angle caused by vertical alignment of the liquid crystal molecule relative to a substrate in application of voltage, is very small. However, it has a problem of large color change relative to viewing angle, because light components transmitting the liquid crystal molecule have dependency of wavelength, or a problem of slow response speed, because the liquid crystal molecule is moved in a horizontal direction relative to a substrate.

On the other hand, in the VA mode, unlike the TN mode, the STN mode or the IPS mode or the like, black display is provided, due to vertical alignment of the liquid crystal molecule relative to a substrate in no application of voltage, and white display is provided, due to horizontal alignment of the liquid crystal molecule relative to a substrate in application of voltage. Because all liquid crystal molecules align completely vertically in no application of voltage, polarized light does not receive influence of the liquid crystal molecule at all, passes through the liquid crystal cell, and is completely blocked by a polarizing plate at the opposite side. For this reason, black image quality becomes very high in principal. In addition, there is little dependency of viewing angle in black display, because the liquid crystal molecule aligned vertically presents the same shape even when viewed from any angle (viewing angle is wide). Still more, there are many merits that response at high speed is possible, from properties of liquid crystal molecule. However, in the case of performing the display of half tone, there is a problem that dependency of viewing angle becomes worse, because the liquid crystal molecule takes an inclined state in the same direction, similarly as in the usual TN mode. The one which has solved a problem of such the dependency of viewing angle in the half tone is an MVA (Multi-domain Vertical Alignment) mode.

The MVA mode is the one called alignment division, and is the one having a plurality of domains where alignment of the liquid crystal molecule differs inside one pixel or inside one sub-pixel. In the MVA mode, there are characteristics that dependency of viewing angle is substantially improved, because light quantity transmitting the liquid crystal cell is made uniform even if viewed from any angle in application of voltage. As technology enabling such the MVA mode, various ones have been known, and among them, a rib-slit method has been known as one of the technology suitable

for the MVA mode (for example, PATENT LITERATURE 1, or the like).

The rib-slit method is the one for providing a triangular prism shaped projection (rib) at the inner surface of a substrate confined the liquid crystal molecule to change the alignment by combination of this shape with electric field effect, and it is called thus because the rib and slit are prepared on a pixel or a sub-pixel. In the rib-slit method, usually, the liquid crystal molecule aligns vertically relative to a substrate in no application of voltage, and, by providing a slope-like rib at the substrate surface, the liquid crystal molecule at the vicinity of the rib is inclined artificially, even in no application of voltage. Because the electric field is applied vertically relative to a substrate, the liquid crystal molecule uniformly inclines in a descending direction of the slope in application of voltage. By providing two slope-like ribs at the substrate surface, the liquid crystal molecule is inclined in two directions separately with the top part of the rib as a boundary. Therefore, a domain of two directions is generated automatically, which enables to attain alignment division required for wider viewing angle. Such the rib-slit method is a method enabling wider viewing angle, by solving a problem of the VA mode that dependency of viewing angle is deteriorated in the case of performing display of the half tone.

On the other hand, other than the rib-slit method, a mask rubbing method has been known as technology suitable for the MVA mode. The mask rubbing method is the one to attain alignment division required for wider viewing angle, by rubbing the liquid crystal alignment film applied on a substrate in a plurality of directions, and by generating a domain in a plurality of directions with inclining the liquid crystal molecule along the rubbing direction (for example, PATENT LITERATURE, 2 or the like).

CITATION LIST

PATENT LITERATURE

PATENT LITERATURE 1: JP-A-11-242225
PATENT LITERATURE 2: JP-A-2005-316363

NON PATENT LITERATURE

NON PATENT LITERATURE 1: Illustrated introduction, Easy understandable basics and mechanism of the newest display technology, ISBN 978-4-7980-2375-5

**SUMMARY OF INVENTION**

**TECHNICAL PROBLEM**

However, the rib-slit method has a problem of deteriorating light utilization efficiency (aperture ratio becomes lower), due to scattering of light on the rib, coming from mechanism providing a rib-slit which does not pass light to the surface of a pixel or a sub-pixel, or a problem of decreasing contrast ratio (black display becomes bright), because of slight leakage of backlight from the slit.

In addition, in a conventional mask rubbing method, the liquid crystal molecule little inclines from a vertical direction relative to a substrate in no application of voltage, although the liquid crystal molecule is inclined by rubbing the liquid crystal alignment film, which raises a problem that generation of a domain in a plurality of directions is difficult.

Under these circumstances, there has been required the development of a liquid crystal display device which can be applied to the MVA mode which enables wider viewing angle, and has solved the problems in the rib-slit method or the conventional mask rubbing method.

The present invention has been made in view of the above situation, and it is intended to provide a composition for a liquid crystal alignment film, a polyamic acid or a polyimide, a liquid crystal alignment film, and a liquid crystal display element or the like, which, even without using a rib-slit, not only enable alignment of the liquid crystal molecule in no application of voltage, but also enable to control pretilt angle of the liquid crystal molecule. Specifically, it is to provide a composition for a liquid crystal alignment film which, in the MVA mode, can hold the liquid crystal molecule in no application of voltage, in a state inclined by several degrees from a vertical direction to a horizontal direction relative to a substrate, as if the rib-slit is present, furthermore enables alignment division by generating a domain by inclining the liquid crystal molecule in a horizontal direction of the substrate in application of voltage; a polyamic acid or a polyimide obtained from the composition; a liquid crystal alignment film composed of the polyamic acid or the polyimide; a method for producing the liquid crystal alignment film, and a liquid crystal display device characterized by being provided with the liquid crystal alignment film, or the like. In addition, it is one of the objects of the present invention to provide also a compound to be used for the composition for the liquid crystal alignment film.

## SOLUTION TO PROBLEM

The present invention relates to a composition for a liquid crystal alignment film comprising components represented by (A)-(D):

(A) a meta-phenylene diamine derivative represented by the general formula [1];

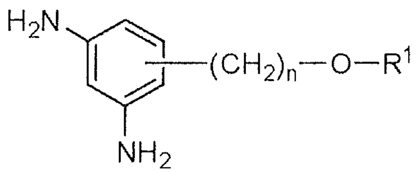

[1]

(wherein $R^1$ represents an alkyl group having 1 to 6 carbon atoms or an acyl group having 2 to 7 carbon atoms, and n represents an integer of 1 to 3.),

(B) a meta-phenylene diamine derivative represented by the general formula [2];

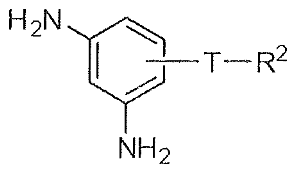

[2]

(wherein $R^2$ represents an alkyl group having 8 to 20 carbon atoms or a group having a steroid skeleton, and T represents an oxygen atom or a carbonyloxy group.),

(C) a para-arylene diamine represented by the general formula [3];

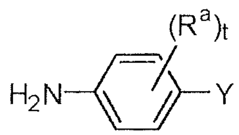

[3]

(wherein Y represents an amino group, an 4-aminophenyl group or an 4-aminophenylmethyl group, t moieties of $R^a$ represent each independently an alkyl group having 1 to 3 carbon atoms, an aryl group having 6 to 10 carbon atoms or an aralkyl group having 7 to 12 carbon atoms, and t represents an integer of 0 to 4. It should be noted that t moieties of $R^a$ may be bound onto a phenyl group in an 4-aminophenyl group or an 4-aminophenylmethyl group represented by Y.),

(D) a tetracarboxylic acid represented by the general formula [4] or a tetracarboxylic acid anhydride represented by the general formula [4'];

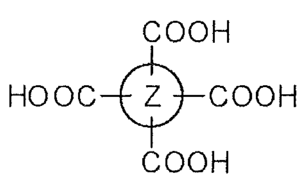

[4]

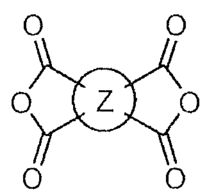

[4']

(wherein Z represents a tetravalent hydrocarbon group.).

In addition, the present invention relates to a polyamic acid or a polyimide obtained by reacting the components represented by the above (A)-(D).

Further, the present invention relates to a liquid crystal aligning agent comprising the above polyamic acid or the polyimide.

Still further, the present invention relates to a liquid crystal alignment film prepared from the above liquid crystal aligning agent.

In addition, the present invention relates to a liquid crystal display element, characterized by being provided with the above liquid crystal alignment film.

Further, the present invention relates to a compound represented by the general formula [1']:

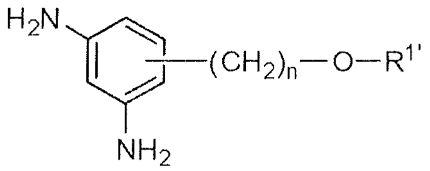

[1']

(wherein $R^{1'}$ represents an acyl group having 2 to 7 carbon atoms, and n represents an integer of 1 to 3.).

Still further, the present invention relates to a method for producing a liquid crystal alignment film for a vertical alignment-type liquid crystal display element, comprising a step for forming a coated film by applying a solution of a polyamic acid or a polyimide obtained by reacting the components represented by the above (A)-(D) onto a support, and a step for performing rubbing processing of the surface of the coated film.

## ADVANTAGEOUS EFFECTS OF INVENTION

The composition for the liquid crystal alignment film of the present invention can provide the liquid crystal alignment film, which can hold the alignment of the liquid crystal molecule in a state inclined by several degrees from a vertical direction to a horizontal direction relative to a substrate, depending on a rubbing direction of the liquid crystal alignment film in no application of voltage, furthermore enables to incline the liquid crystal molecule in a rubbing direction of the liquid crystal alignment film in application of voltage, in other words, the liquid crystal alignment film which is possible to control pretilt angle of the liquid crystal molecule in no application of voltage. Application of such the liquid crystal alignment film to the MVA mode enables to attain same wider viewing angle as the rib-slit method. In addition, in the liquid crystal display element provided with such the liquid crystal alignment film, such the problem itself does not present that light utilization efficiency is poor in the rib-slit method (aperture ratio is low) or contrast ratio is low, because the rib itself is not required. That is, the liquid crystal display element provided with the liquid crystal alignment film obtained from the composition for the liquid crystal alignment film of the present invention can attain wider viewing angle even in display of half tone, and further exerts effect of being able to prepare the liquid crystal display element more simply and conveniently, due to no requirement of the rib itself.

Further, the method for producing of the present invention is a method which enables to produce the liquid crystal alignment film, which can control pretilt angle of the liquid crystal molecule in no application of voltage, in a range of 75 to 87 degree, and the liquid crystal display element provided with such the liquid crystal alignment film can attain wider viewing angle, and further, has good light utilization efficiency (aperture ratio is high), in addition, can make contrast ratio better, therefore it exerts effect of being able to provide a high quality liquid crystal display element in such the point.

That is, in a liquid crystal alignment film of a conventional VA mode, the liquid crystal alignment film is used with the aim of making the liquid crystal molecule align in a vertical direction (make vertically stand) relative to a substrate in no application of voltage. Accordingly, there have been performed many investigations on a compound for expressing pretilt angle of the liquid crystal molecule at 90 degree (making it stand) or introduction amount of the compound. On the other hand, the present inventors found for the first time that, in the liquid crystal alignment film prepared from the composition including the meta-phenylene diamine derivative represented by the above general formula [1], the liquid crystal molecule inclines depending on a rubbing direction of the alignment film. That is, in the VA mode, there has not been an idea of intending to incline the liquid crystal molecule by the alignment film up to now, and the present inventors considered, from this fact, that the liquid crystal alignment film suitable for alignment division can be provided, and started the investigation thereon and has thus completed the present invention.

## DESCRIPTION OF EMBODIMENTS

A composition for a liquid crystal alignment film of the present invention is the one characterized by comprising components represented by the following (A)-(D):

(A) a meta-phenylene diamine derivative represented by the general formula [1];

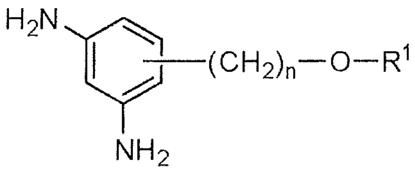

[1]

(wherein $R^1$ represents an alkyl group having 1 to 6 carbon atoms or an acyl group having 2 to 7 carbon atoms, and n represents an integer of 1 to 3.),

(B) a meta-phenylene diamine derivative represented by the general formula [2];

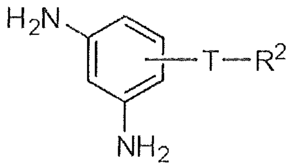

[2]

(wherein $R^2$ represents an alkyl group having 8 to 20 carbon atoms or a group having a steroid skeleton, and T represents an oxygen atom or a carbonyloxy group.),

(C) a para-arylene diamine represented by the general formula [3];

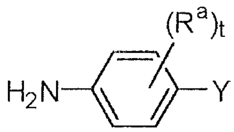

[3]

(wherein Y represents an amino group, an 4-aminophenyl group, or an 4-aminophenylmethyl group, t moieties of $R^a$ represent each independently an alkyl group having 1 to 3 carbon atoms, an aryl group having 6 to 10 carbon atoms, or an aralkyl group having 7 to 12 carbon atoms, and t represents an integer of 0 to 4. It should be noted that t moieties of $R^a$ may be bound onto a phenyl group in an 4-aminophenyl group or an 4-aminophenylmethyl group represented by Y.),

(D) a tetracarboxylic acid represented by the general formula [4] or a tetracarboxylic acid anhydride represented by the general formula [4'];

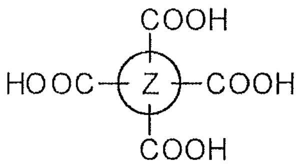

[4]

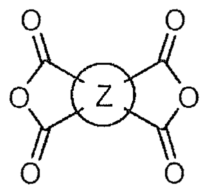

[4']

(wherein Z represents a tetravalent hydrocarbon group.).

A polyamic acid or a polyimide of the present invention is the one characterized by being obtained by reacting the components represented by the above (A)-(D).

A liquid crystal aligning agent of the present invention is the one characterized by comprising the above polyamic acid or the polyimide.

A liquid crystal alignment film not having a rib of the present invention is the one characterized by being prepared from the above liquid crystal aligning agent.

A liquid crystal display element of the present invention is the one characterized by being provided with the above liquid crystal alignment film.

A compound of the present invention is the one represented by the following general formula [1']:

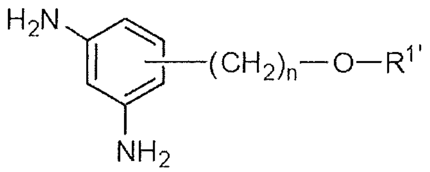

[1']

(wherein $R^{1'}$ represents an acyl group having 2 to 7 carbon atoms, and n represents an integer of 1 to 3.).

A method for producing a liquid crystal alignment film for a vertical alignment-type liquid crystal display element of the present invention is the one characterized by comprising a step for forming a coated film by applying a solution of a polyamic acid or a polyimide obtained by reacting the components represented by the above (A)-(D) onto a support, and a step for performing rubbing processing of the surface of the coated film.

In the meta-phenylene diamine derivative represented by the general formula [1], an alkyl group having 1 to 6 carbon atoms represented by $R^1$ may be any alkyl groups of linear, branched and cyclic, and specifically includes, for example, a methyl group, an ethyl group, a n-propyl group, an isopropyl group, a n-butyl group, an isobutyl group, a sec-butyl group, a tert-butyl group, a cyclobutyl group, a n-pentyl group, an isopentyl group, a sec-pentyl group, a tert-pentyl group, a neopentyl group, a 2-methylbutyl group, a 1,2-dimethylpropyl group, an 1-ethylpropyl group, a cyclopentyl group, a n-hexyl group, an isohexyl group, a sec-hexyl group, a tert-hexyl group, a neohexyl group, a 2-methylpentyl group, a 1,2-dimethylbutyl group, a 2,3-dimethylbutyl group, an 1-ethylbutyl group, a cyclohexyl group, and the like. Among these alkyl groups, a linear or branched alkyl group having 1 to 4 carbon atoms is preferable, specifically, it includes a methyl group, an ethyl group, a n-propyl group, an isopropyl group, a n-butyl group, an isobutyl group, a sec-butyl group, or a tert-butyl group, as a preferable alkyl group.

In the meta-phenylene diamine derivative represented by the general formula [1] and the compound represented by the general formula [1'], an acyl group having 2 to 7 carbon atoms represented by $R^1$ and $R^{1'}$ may be any acyl group of a linear, branched and cyclic, and specifically includes, for example, an acetyl group (an ethanoyl group), a propionyl group (a propanoyl group), a butyryl group (a butanoyl group), an isobutyryl group, a valeryl group (a pentanoyl group), an isovaleryl group, a pivaloyl group, a cyclobutanecarbonyl group, a caproyl group (a hexanoyl group), a cyclopentanecarbonyl group, an enanthyl group (a heptanoyl group), a cyclohexanecarbonyl group, and the like. Among these acyl groups, a linear or branched acyl group having 2 to 5 carbon atoms is preferable, specifically it includes an acetyl group (an ethanoyl group), a propionyl group (a propanoyl group), a butyryl group (a butanoyl group), an isobutyryl group, a valeryl group (a pentanoyl group), an isovaleryl group, and a pivaloyl group, as a preferable acyl group.

In the meta-phenylene diamine derivative represented by the general formula [1] and the compound represented by the general formula [1'], n includes an integer of 1 to 3, and among them, 2 is more preferable.

In the meta-phenylene diamine derivative represented by the general formula [1] and the compound represented by the general formula [1'], as a methylene group binding to a diaminophenyl group, in the case that the a binding position of the methylene group for a benzene ring is assumed to be 1-position, the one where two amino groups are bound at the position of 2-position and 4-position, and the one where two amino groups are bound at the position of 3-position and 5-position are preferable, and among them, the one where two amino groups are bound at the position of 2-position and 4-position is more preferable.

In the meta-phenylene diamine derivative represented by the general formula [2], an alkyl group having 8 to 20 carbon atoms represented by $R^2$ may be any alkyl group of linear, branched and cyclic, and specifically includes, for example, a n-octyl group, an isooctyl group, a sec-octyl group, a tert-octyl group, a neooctyl group, an 2-ethylhexyl group, a cyclooctyl group, a n-nonyl group, an isononyl group, a sec-nonyl group, a tert-nonyl group, a neononyl group, a cyclononyl group, a n-decyl group, an isodecyl group, a sec-decyl group, a tert-decyl group, a neodecyl group, a cyclodecyl group, a n-undecyl group, a cycloundecyl group, a n-dodecyl group, a cyclododecyl group, a n-tridecyl group, a cyclotridecyl group, a n-tetradecyl group, a cyclotetradecyl group, a n-pentadecyl group, a cyclopentadecyl group, a n-hexadecyl group, a cyclohexadecyl group, a n-heptadecyl group, a cycloheptadecyl group, a n-octadecyl group, a cyclooctadecyl group, a n-nonadecyl group, a cyclononadecyl group, a n-icosyl group, a cycloicosyl group, a bornyl group (bornane-$\chi$-yl group), an adamantyl group, a methyladamantyl group, a menthyl group (a mentha-$\chi$-yl group), a decahydronaphthyl group, and the like. Among these alkyl groups, a linear alkyl group having 12 to 20 carbon atoms is preferable, and specifically includes a n-dodecyl group, a n-tridecyl group, a n-tetradecyl group, a n-pentadecyl group, a n-hexadecyl group, a n-heptadecyl group, a n-octadecyl group, a n-nonadecyl group, and a n-icosyl group, as a preferable alkyl group.

In the meta-phenylene diamine derivative represented by the general formula [2], as the group having a steroid skeleton represented by $R^2$, it includes a monovalent organic group which has a cyclopentahydrophenanthrene skeleton as a basic skeleton, and 3-position thereof is bound to T in the general formula [2]. As specific example thereof, there is included, for example, a group represented by the following chemical formula, that is, a cholestanyl group, a cholesteryl group, an ergostanyl group, a spirostanyl group, and the like, but it is not limited to these specific examples, and is not

especially limited, as long as it has a cyclopentahydrophenanthrene skeleton as a basic skeleton. Among these groups having a steroid skeleton, a β-cholestanyl group is cited as the preferable one.

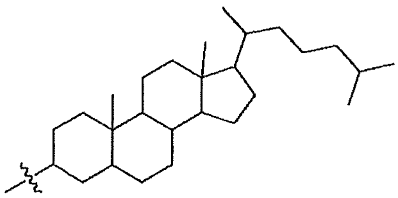

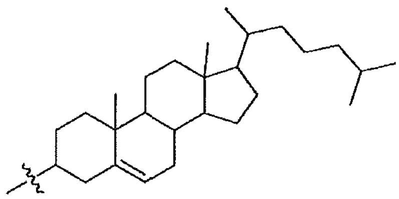

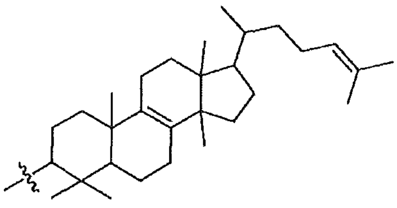

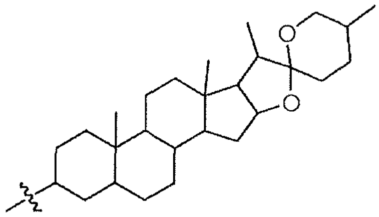

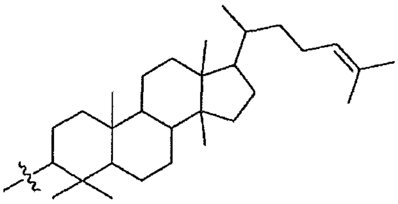

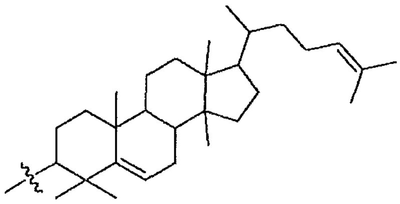

In the meta-phenylene diamine derivative represented by the general formula [2], a divalent group represented by T represents includes an oxygen atom (-O-), or a carbonyloxy group (-COO-). In the case where T is an oxygen atom, it means the one where a benzene ring and $R^2$ are bound via the oxygen atom (ether bond), and in the case when T is carbonyloxy group, it means the one where a benzene ring and $R^2$ are bound via the carbonyloxy group (ester bond). It should be noted that, in the case where T is a carbonyloxy group, a carbon atom of the carbonyloxy group binds to a benzene ring, and an oxygen atom of the carbonyloxy group binds to $R^2$.

In the meta-phenylene diamine derivative represented by the general formula [2], in the case that a carbon atom on a benzene ring to which T binds, is numbered as 1-position, as a binding position of T binding to a diaminophenyl group, such a case is preferable where carbon atoms bound with two amino groups are numbered as 3-position and 5-position respectively.

In the para-arylene diamine represented by the general formula [3], an alkyl group having 1 to 3 carbon atoms represented by $R^a$ may be any alkyl group of linear and branched, and specifically includes, for example, a methyl group, an ethyl group, a n-propyl group, an isopropyl group, and the like. Among these alkyl groups, a methyl group, which is an alkyl group having 1 carbon atom, is included as a preferable alkyl group.

In the para-arylene diamine represented by the general formula [3], an aryl group having 6 to 10 carbon atoms represented by $R^a$ may be any aryl group of monocyclic and condensed polycyclic, and specifically includes, for example, a phenyl group, or a naphthyl group, and the like. Among these aryl groups, a phenyl group, which is an aryl group having 6 carbon atoms, is included as a preferable aryl group.

In the para-arylene diamine represented by the general formula [3], an aralkyl group having 7 to 12 carbon atoms represented by $R^a$ may be any aralkyl group of a monocyclic and condensed polycyclic, and specifically includes, for example, a benzyl group, a phenethyl group, a methylbenzyl group, a phenylpropyl group, a 1-methylphenylethyl group, a phenylbutyl group, a 2-methylphenylpropyl group, a tetrahydronaphthyl group, a naphthylmethyl group, a naphthylethyl group, and the like. Among these aralkyl groups, a benzyl group, which is an aralkyl group having 7 carbon atoms, is included as a preferable aralkyl group.

In the para-arylene diamine represented by the general formula [3], t includes an integer of 0 to 4, and among them, 0 is more preferable.

In the para-arylene diamine represented by the general formula [3], a monovalent organic group represented by Y includes an amino group, an 4-aminophenyl group, an 4-aminophenylmethyl group, and the like, and among them, an amino group is preferable.

In the para-arylene diamine represented by the general formula [3], t moieties of $R^a$ may be same or different from each other, and in the case where t is an integer of 1 to 4 and furthermore, a monovalent organic group represented

by Y is an 4-aminophenyl group or an 4-aminophenylmethyl group, a part or all of t moieties of $R^a$ may be a substituted group in an 4-aminophenyl group or an 4-aminophenylmethyl group.

In a tetracarboxylic acid represented by the general formula [4] or a tetracarboxylic acid anhydride represented by the general formula [4'], as a tetravalent hydrocarbon group represented by Z, it includes the one represented by, for example, the following chemical formulae [4-A]-[4-i]:

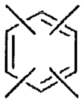 [4-A] 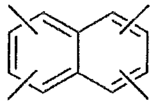 [4-B] 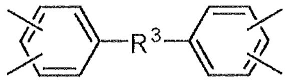 [4-C]

(wherein $R^3$ represents a bond, an oxygen atom, a methylene group, a perfluorodimethylmethylene group, a carbonyl group or a sulfonyl group.);

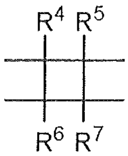 [4-D] 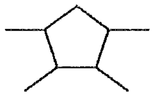 [4-E] 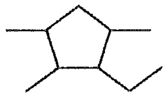 [4-F]

(wherein $R^4$ to $R^7$ independently represent a hydrogen atom or an alkyl group having 1 to 3 carbon atoms.);

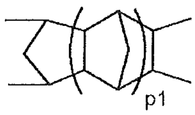 [4-G] 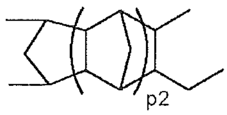 [4-H]

(wherein p1 and p2 independently represent 0 or 1.);

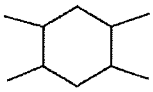 [4-I] 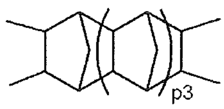 [4-J] 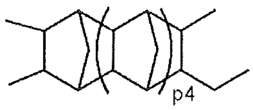 [4-K]

(wherein p3 and p4 independently represent 0 or 1.);

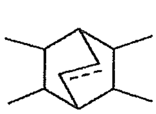 [4-L] 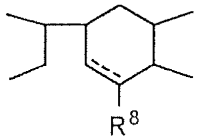 [4-M] 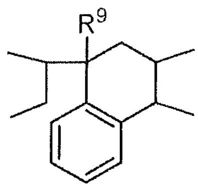 [4-N]

(wherein $R^8$ and $R^9$ independently represent a hydrogen atom or an alkyl group having 1 to 3 carbon atoms, and a bond represented by a solid line and a dotted line means that it may be any bond of a single bond and a double bond.);

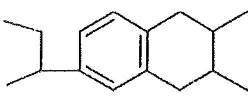 [4-O] 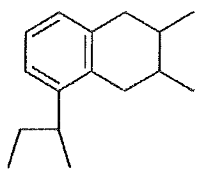 [4-P] 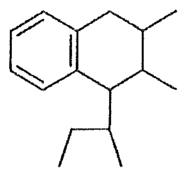 [4-Q]

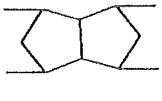 [4-R] 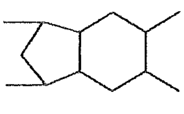 [4-S] 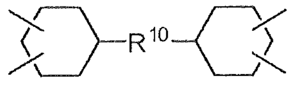 [4-T]

(wherein $R^{10}$ represents a bond, an oxygen atom, a sulfur atom, a methylene group or a sulfonyl group.);

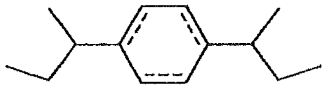 [4-U] 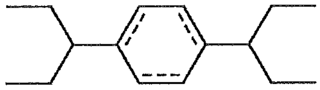 [4-V]

(wherein a bond represented by a solid line and a dotted line means that it may be any bond of a single bond and a double bond.);

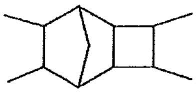 [4-W] 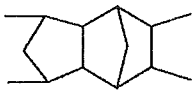 [4-X] 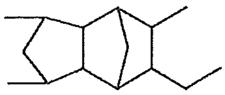 [4-Y]

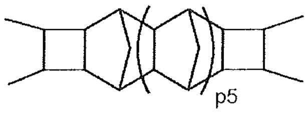 [4-Z] 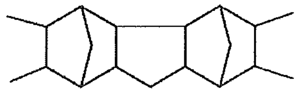 [4-a]

(wherein p5 represents 0 or 1.);

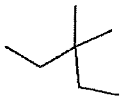 [4-b] 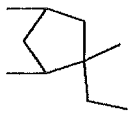 [4-c] 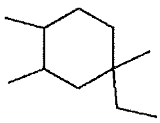 [4-d]

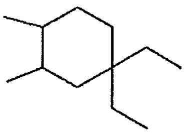 [4-e] 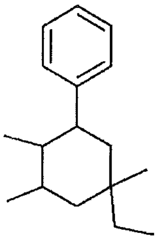 [4-f] 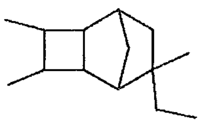 [4-g]

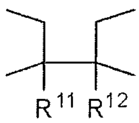 [4-h] 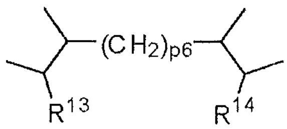 [4-i]

(wherein $R^{11}$ to $R^{14}$ independently represent a hydrogen atom or an alkyl group having 1 to 3 carbon atoms, and p6 represents an integer of 8 to 20.).

In the chemical formula [4-C], $R^3$ includes a bond, an oxygen atom, a methylene group, a perfluorodimethylmethylene group, a carbonyl group and a sulfonyl group, and among them, an oxygen atom and a methylene group are preferable, and among them, a methylene group is more preferable.

In the chemical formulae [4-D], [4-M], [4-N], [4-h] and [4-i], an alkyl group having 1 to 3 carbon atoms represented by $R^4$ to $R^9$ and $R^{11}$ to $R^{14}$ may be any alkyl group of linear and branched, and specifically includes, for example, a methyl group, an ethyl group, a n-propyl group and an isopropyl group. Among these alkyl groups, a methyl group, which is an alkyl group having 1 carbon atoms, is included as a preferable alkyl group.

As $R^4$ to $R^9$ and $R^{11}$ to $R^{14}$ in the chemical formulae [4-D], [4-M], [4-N], [4-h] and [4-i], it includes a hydrogen atom and an alkyl group having 1 to 3 carbon atoms, and among them, a hydrogen atom is preferable.

In the chemical formulae [4-G], [4-H], [4-J], [4-K] and [4-Z], p1 to p5 includes an integer of 0 or 1, and among them, 0 is more preferable.

In the chemical formula [4-T], $R^{10}$ includes a bond, an oxygen atom, a sulfur atom, a methylene group or a sulfonyl group, and among them, an oxygen atom and a methylene group are preferable, and among them, a methylene group is more preferable.

In the chemical formula [4-i], p6 includes an integer of 8 to 20.

In the composition for the liquid crystal alignment film of the present invention, the meta-phenylene diamine derivative represented by the above general formula [1] is used mainly to control pretilt angle of a liquid crystal molecule,

in the liquid crystal alignment film prepared from the composition.

The meta-phenylene diamine derivative represented by the general formula [1] is largely classified to a meta-phenylene diamine derivative represented by the general formula [1'] and a meta-phenylene diamine derivative represented by the general formula [1"]:

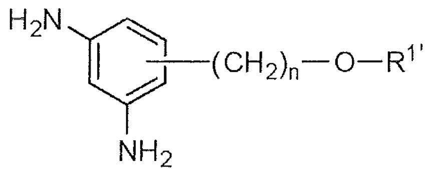

[1']

(wherein $R^{1'}$ represents an acyl group having 2 to 7 carbon atoms, and n represents an integer of 1 to 3.);

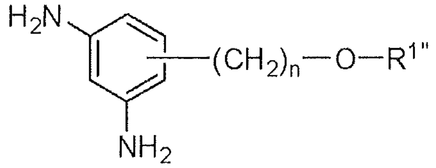

[1"]

(wherein $R^{1''}$ represents an alkyl group having 1 to 6 carbon atoms, and n represents an integer of 1 to 3.).

In the compounds represented by the general formula [1"], an alkyl group having 1 to 6 carbon atoms represented by $R^{1''}$ may be any alkyl group of linear, branched and cyclic, and specifically includes, for example, a methyl group, an ethyl group, a n-propyl group, an isopropyl group, a n-butyl group, an isobutyl group, a sec-butyl group, a tert-butyl group, a cyclobutyl group, a n-pentyl group, an isopentyl group, a sec-pentyl group, a tert-pentyl group, a neopentyl group, a 2-methylbutyl group, a 1,2-dimethylpropyl group, an 1-ethylpropyl group, a cyclopentyl group, a n-hexyl group, an isohexyl group, a sec-hexyl group, a tert-hexyl group, a neohexyl group, a 2-methylpentyl group, a 1,2-dimethylbutyl group, a 2,3-dimethylbutyl group, an 1-ethylbutyl group, a cyclohexyl group, and the like. Among these alkyl groups, a linear or branched alkyl group having 1 to 4 carbon atoms is preferable, and specifically it includes a methyl group, an ethyl group, a n-propyl group, an isopropyl group, a n-butyl group, an isobutyl group, a sec-butyl group, and a tert-butyl group, as a preferable alkyl group.

Specific examples of meta-phenylene diamine derivatives represented by the general formula [1'] include, for example, 2,4-diaminobenzyl acetate, 2,4-diaminobenzyl propionate, 2,4-diaminobenzyl butyrate, 2,4-diaminobenzyl pentylate, 2,4-diaminobenzyl hexylate, 2,4-diaminobenzyl heptylate, 2,4-diaminophenethyl acetate, 2,4-diaminophenethyl propionate, 2,4-diaminophenethyl butyrate, 2,4-diaminophenethyl pentylate, 2,4-diaminophenethyl hexylate, 2,4-diaminophenethyl heptylate, 2,4-diaminophenylpropyl acetate, 2,4-diaminophenylpropyl propionate, 2,4-diaminophenylpropyl butyrate, 2,4-diaminophenylpropyl pentylate, 2,4-diaminophenylpropyl hexylate, 2,4-diaminophenylpropyl heptylate, 3,5-diaminobenzyl acetate, 3,5-diaminobenzyl propionate, 3,5-diaminobenzyl butyrate, 3,5-diaminobenzyl pentylate, 3,5-diaminobenzyl hexylate, 3,5-diaminobenzyl heptylate, 3,5-diaminophenethyl acetate, 3,5-diaminophenethyl propionate, 3,5-diaminophenethyl butyrate, 3,5-diaminophenethyl pentylate, 3,5-diaminophenethyl hexylate, 3,5-diaminophenethyl heptylate, 3,5-diaminophenylpropyl acetate, 3,5-diaminophenylpropyl propionate, 3,5-diaminophenylpropyl butyrate, 3,5-diaminophenylpropyl pentylate, 3,5-diaminophenylpropyl hexylate, 3,5-diaminophenylpropyl heptylate, and the like. Among these meta-phenylene diamine derivatives represented by the general formula [1'], 2,4-diaminophenethyl acetate, 2,4-diaminophenethyl propionate and 2,4-diaminophenethyl butyrate are included as the preferable ones. Because 2,4-diaminophenethyl acetate, 2,4-diaminophenethyl propionate and 2,4-diaminophenethyl butyrate have high polymerization activity, they easily react with a tetracarboxylic acid or an anhydride of the tetracarboxylic acid, and in addition, a liquid crystal alignment film prepared from a composition including any of 2,4-diaminophenethyl acetate, 2,4-diaminophenethyl propionate and 2,4-diaminophenethyl butyrate is included as the preferable one from the viewpoint that a liquid crystal molecule inclines in suitable angle relative to a substrate from a vertical direction to a horizontal direction by rubbing, and a liquid crystal display element containing the liquid crystal molecule having an optimum pretilt angle can be obtained, as compared with a liquid crystal alignment film containing other meta-phenylene diamine derivatives.

Specific examples of the meta-phenylene diamine derivatives represented by the general formula [1"] include, for example, 2,4-diaminobenzyl methyl ether, 2,4-diaminobenzyl ethyl ether, 2,4-diaminobenzyl propyl ether, 2,4-diaminobenzyl butyl ether, 2,4-diaminobenzyl pentyl ether, 2,4-diaminobenzyl hexyl ether, 2,4-diaminophenethyl methyl ether, 2,4-diaminophenethyl ethyl ether, 2,4-diaminophenethyl propyl ether, 2,4-diaminophenethyl butyl ether, 2,4-diaminophenethyl pentyl ether, 2,4-diaminophenethyl hexyl ether, 2,4-diaminophenylpropyl methyl ether, 2,4-diaminophenylpropyl ethyl ether, 2,4-diaminophenylpropyl propyl ether, 2,4-diaminophenylpropyl butyl ether, 2,4-diaminophenylpropyl pentyl ether, 2,4-diaminophenylpropyl hexyl ether, 3,5-diaminobenzyl methyl ether, 3,5-diaminobenzyl ethyl ether, 3,5-diaminobenzyl propyl ether, 3,5-diaminobenzyl butyl ether, 3,5-diaminobenzyl pentyl ether, 3,5-diaminobenzyl hexyl ether, 3,5-diaminophenethyl methyl ether, 3,5-diaminophenethyl ethyl ether, 3,5-diaminophenethyl propyl ether, 3,5-diaminophenethyl butyl ether, 3,5-diaminophenethyl pentyl ether, 3,5-diaminophenethyl hexyl ether, 3,5-diaminophenylpropyl methyl ether, 3,5-diaminophenylpropyl ethyl ether, 3,5-diaminophenylpropyl propyl ether, 3,5-diaminophenylpropyl butyl ether, 3,5-diaminophenylpropyl pentyl ether, 3,5-diaminophenylpropyl hexyl ether, and the like. Among these meta-phenylene diamine derivatives represented by the general formula [1"], 2,4-diaminophenethyl methyl ether, 2,4-diaminophenethyl ethyl ether, 2,4-diaminophenethyl propyl ether and 2,4-diaminophenethyl butyl ether are included as preferable one, and among them, 2,4-diaminophenethyl methyl ether are included as the more preferable ones. Because 2,4-diaminophenethyl methyl ether has high polymerization activity, it easily reacts with a tetracarboxylic acid or an anhydride of the tetracarboxylic acid, and in addition, a liquid crystal alignment film prepared from a composition including 2,4-diaminophenethyl methyl ether is included as the preferable one from the viewpoint that a liquid crystal molecule inclines in suitable angle relative to a substrate from a vertical direction to a horizontal direction by rubbing, and a liquid crystal display element containing the liquid crystal molecule having optimum pretilt angle can be obtained, as compared with a liquid crystal alignment film containing other meta-phenylene diamine derivatives.

As these meta-phenylene diamine derivatives, those synthesized appropriately by a common method may be used, or the commercially available one may also be used. In addition, these meta-phenylene diamine derivatives may be used alone as one kind, or a plurality of kinds may be used in combination, as appropriate.

These meta-phenylene diamine derivatives may be synthesized appropriately by adopting a general method. As a specific synthesis method, for example, after nitrating commercially available phenethyl acetate or a phenethyl alkyl ether according to a common method, by reducing the relevant nitro group according to a common method, 2,4-diaminophenethyl acetate or a 2,4-diaminophenethyl alkyl ether can be synthesized. In addition, as other synthetic methods, for example, after reducing 2,4-dinitrophenethyl acetate to obtain 2,4-dinitrophenethyl alcohol, by acylating it with an acylating agent having desired carbon atoms, a 2,4-dinitrophenethyl alkylate can be synthesized. Then, by reducing a nitro group of the 2,4-dinitrophenethyl alkylate, a 2,4-diaminophenethyl alkylate can be synthesized. Further, as other methods, for example, after nitrating commercially available benzyl alkyl ether according to a common method, by reducing the relevant nitro group, a 2,4-diaminobenzyl alkyl ether can be synthesized.

The liquid crystal alignment film prepared from the composition containing the meta-phenylene diamine derivative represented by such the general formula [1] is the one which enables to make a liquid crystal molecule aligned in no application of voltage, depending on a rubbing direction, as well as can maintain pretilt angle of the liquid crystal molecule at 75 to 87 degree relative to a substrate. That is, the meta-phenylene diamine derivative represented by the general formula [1] is a compound effective for attaining control of pretilt angle by making the liquid crystal molecule aligned in no application of voltage.

In the composition for the liquid crystal alignment film of the present invention, the meta-phenylene diamine derivative represented by the above general formula [2] is used mainly to exert pretilt angle of the liquid crystal molecule, in the liquid crystal alignment film prepared from the composition. In more specifically, the meta-phenylene diamine derivative represented by the above general formula [2] is the one to be used for pretilt angle of the liquid crystal molecule to make closer to a vertical direction (90 degree) relative to a substrate.

As specific examples of the meta-phenylene diamine derivatives represented by the general formula [2], for example, the one represented by the following chemical formula is included, and among them, β-cholestanyl 3,5-diaminobenzoate, which is a β-type of a compound represented by the formula [2-1], β-cholestanyl 2,4-diaminobenzoate, which is a β-type of a compound represented by the formula [2-7], β-cholestanyl 2,4-diaminophenol, which is a β-type of a compound represented by the formula [2-13], are included as the preferable ones. β-Cholestanyl 3,5-diaminobenzoate, β-cholestanyl 2,4-diaminobenzoate and β-cholestanyl 2,4-diaminophenol are included as the preferable ones in view of not only easy availability but also high expression characteristics of pretilt angle.

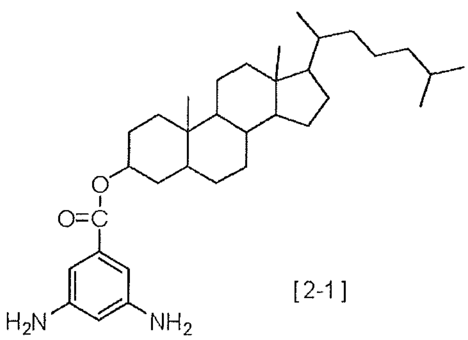
[2-1]

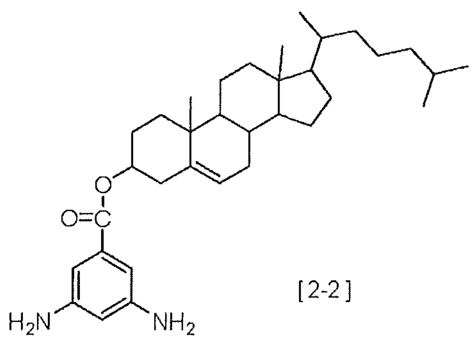
[2-2]

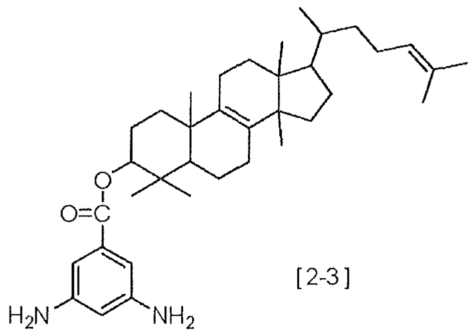

[2-3]

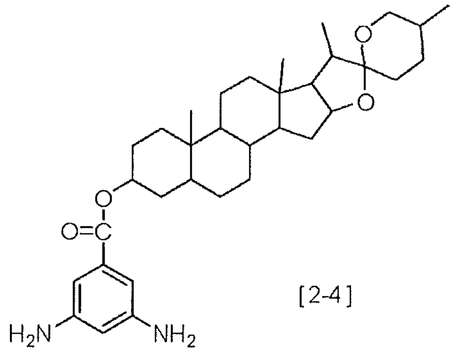

[2-4]

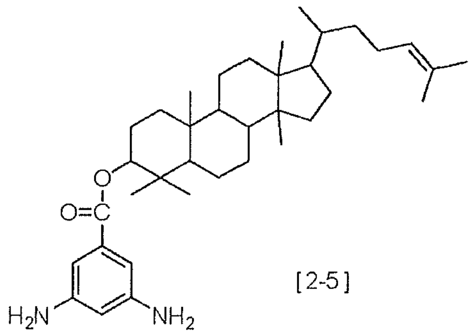

[2-5]

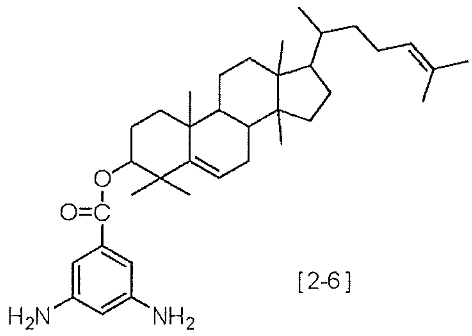

[2-6]

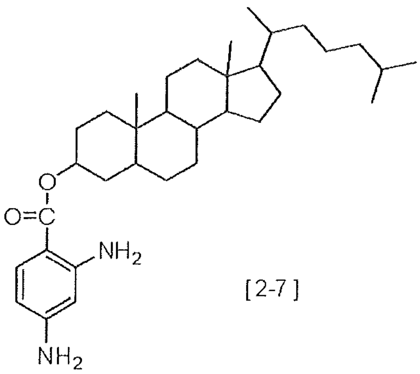

[2-7]

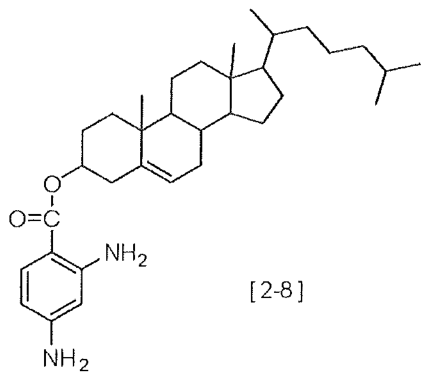

[2-8]

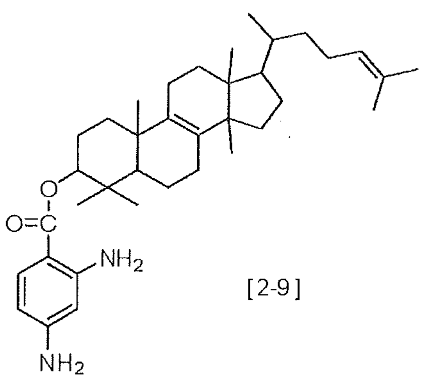

[2-9]

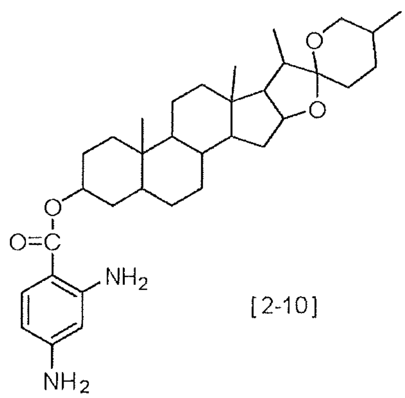

[2-10]

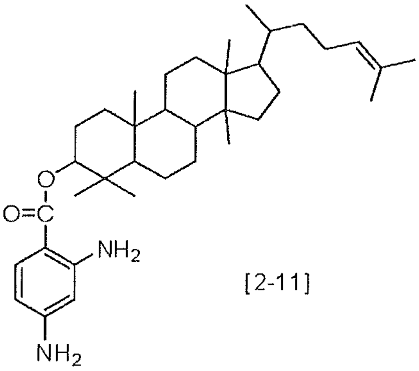

[2-11]

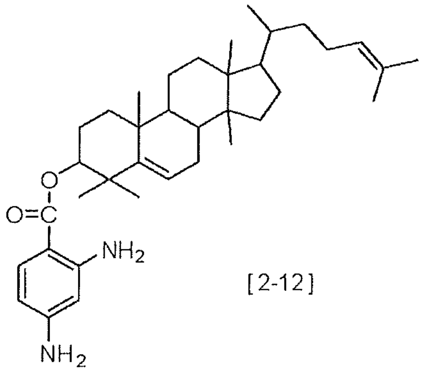

[2-12]

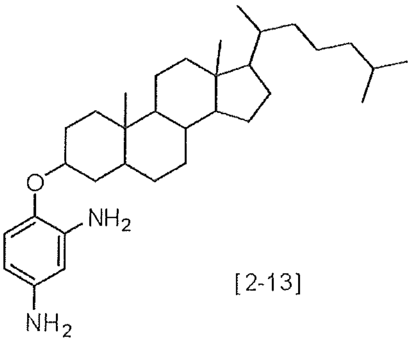

[2-13]

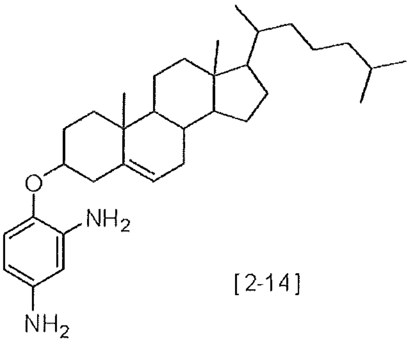

[2-14]

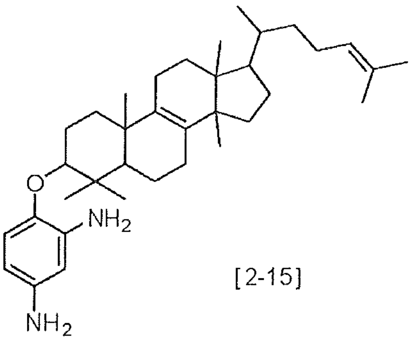

[2-15]

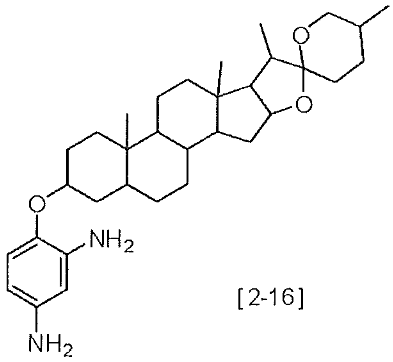

[2-16]

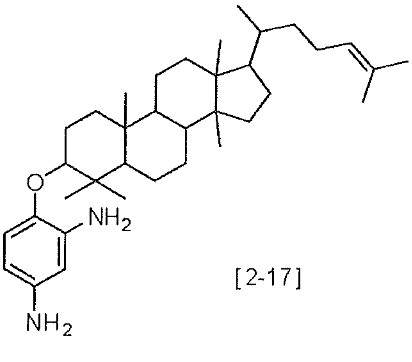

[2-17]

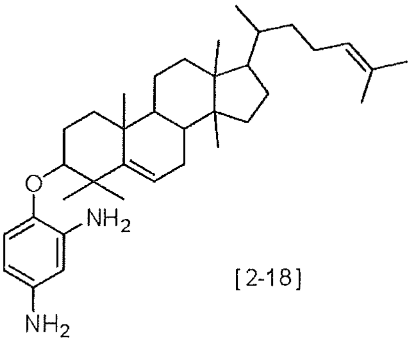

[2-18]

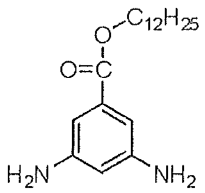

[2-19]

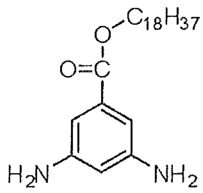

[2-20]

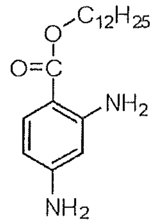

[2-21]

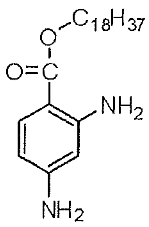

[2-22]

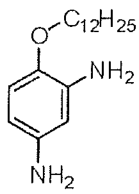

[2-23]

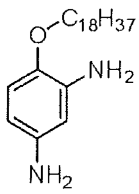

[2-24]

As these meta-phenylene diamine derivatives, those synthesized appropriately by a common method may be used, or the commercially available one may also be used. In addition, these meta-phenylene diamine derivatives may be used alone as one kind, or a plurality of kinds may be used in combination, as appropriate.

These meta-phenylene diamine derivatives may be synthesized appropriately by adopting a general method. As a specific synthesis method, for example, after obtaining cholestanyl 3,5-dinitrobenzoate by reacting 3,5-dintrobenzoyl chloride with cholestanol, under basic condition of, for example, triethylamine, or the like, cholestanyl 3,5-diaminobenzoate can be synthesized by hydrogen reduction using palladium / carbon as a catalyst.

In the composition for the liquid crystal alignment film of the present invention, the para-arylene diamine represented by the above general formula [3] is mainly used to obtain a high quality alignment film, in the liquid crystal alignment film prepared from the composition. In more specifically, the para-arylene diamine represented by the above general formula [3] is the one which is used to obtain a liquid crystal alignment film having an optimum film thickness and high durability.

As specific examples of the para-arylene diamine represented by the general formula [3], for example, the one represented by the following chemical formula is included.

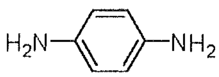

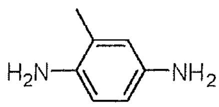

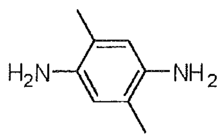

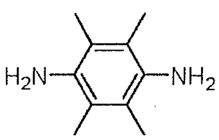

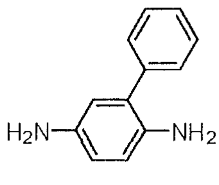

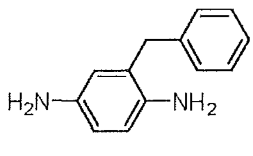

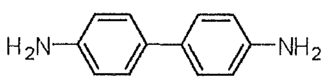

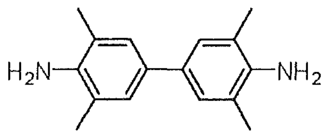

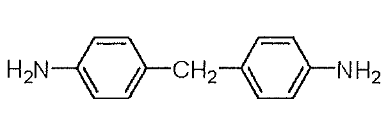

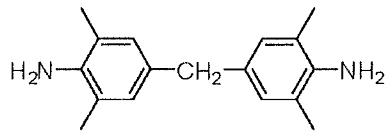

Among these para-arylene diamines represented by such the chemical formula, para-arylene diamines represented by the general formula [3'] are preferable.

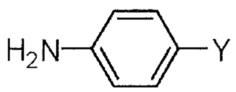 [3']

(wherein Y represents an amino group, an 4-aminophenyl group or an 4-aminophenylmethyl group)

As these para-arylene diamines, those synthesized appropriately by a common method may be used, or the commercially available one may also be used. In addition, these para-arylene diamines may be used alone as one kind, or a plurality of kinds may be used in combination, as appropriate.

In the composition for forming the liquid crystal alignment film of the present invention, a tetracarboxylic acid represented by the above general formula [4] or a tetracarboxylic acid anhydride represented by the general formula [4'] is the one to be used to obtain a polyamic acid or a polyimide polymer by reacting with a diamine represented by the above general formulae [1]-[3], in preparing the liquid crystal alignment film from the composition.

As specific examples of the tetracarboxylic acid represented by the general formula [4] or the tetracarboxylic acid anhydride represented by the general formula [4'], those derived from the above formulae [4-A]-[4-i] are included, and specifically, for example, the one represented by the following chemical formula is included.

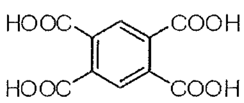

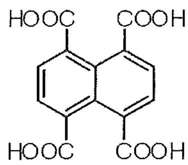

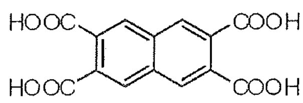

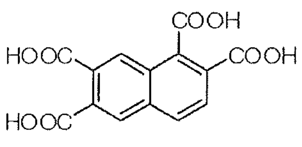

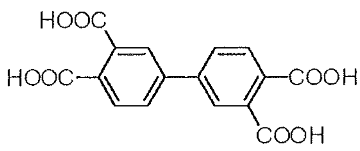

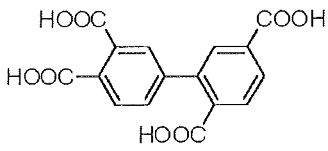

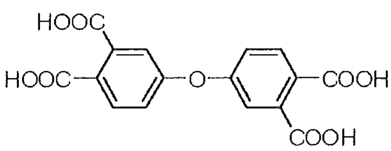

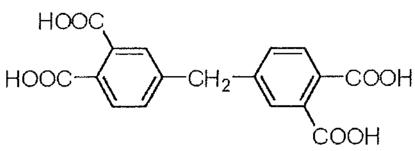

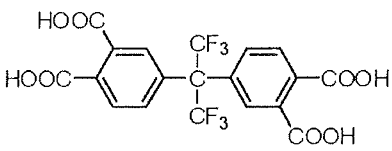

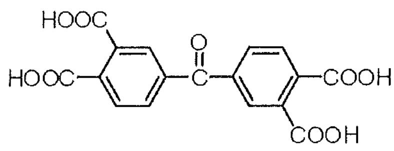

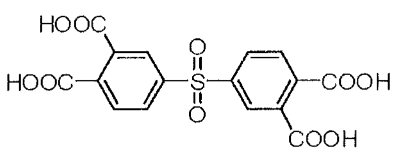

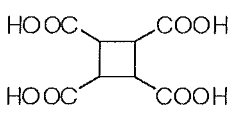
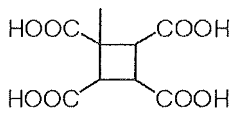
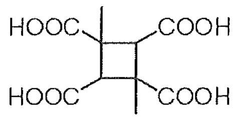

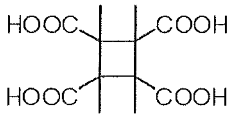
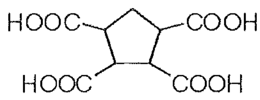
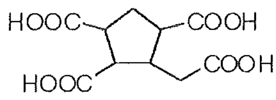

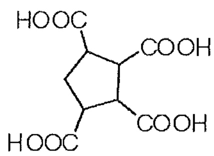
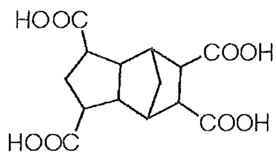

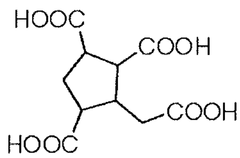
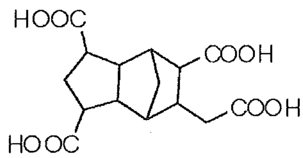

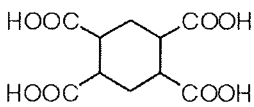
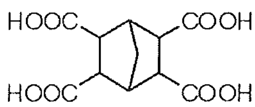
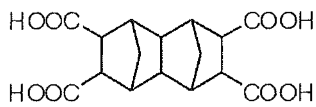

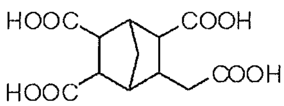
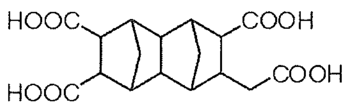

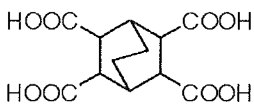
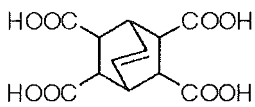
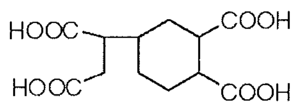

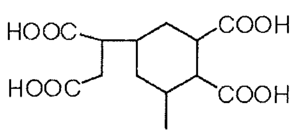
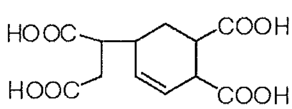
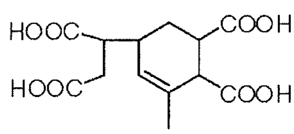

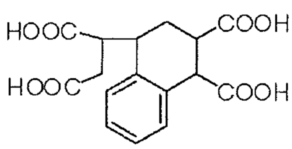
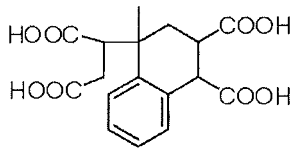

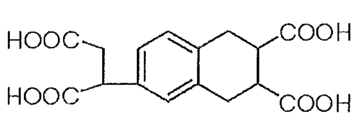
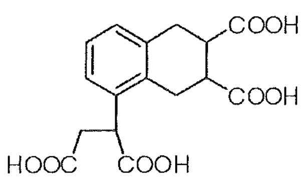
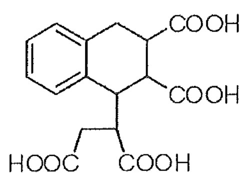

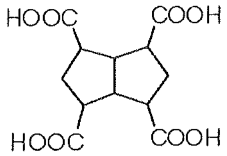 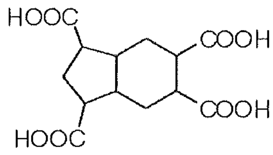 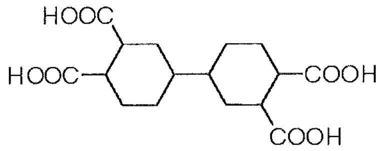

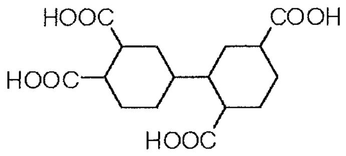 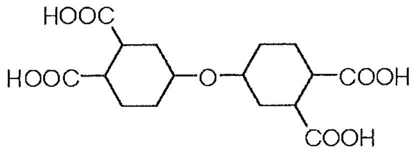

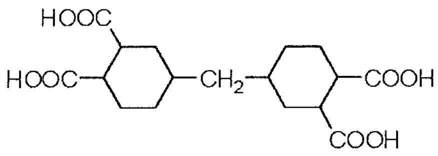 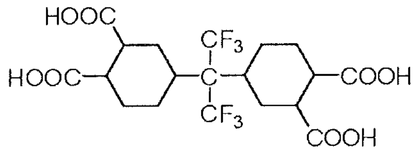

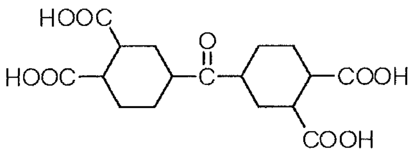 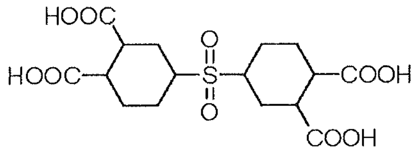

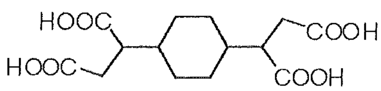 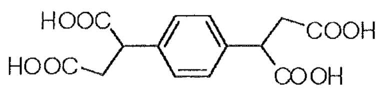

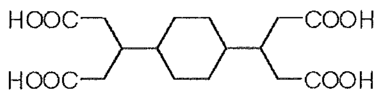 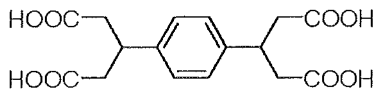

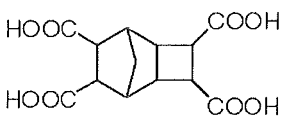 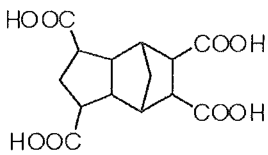 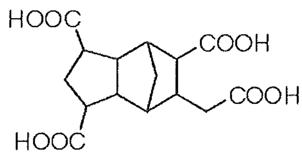

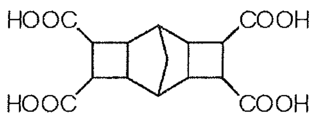 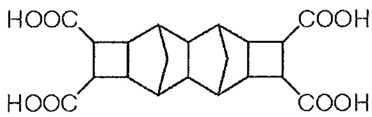

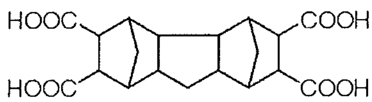

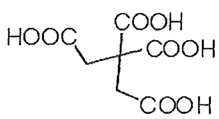 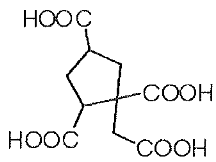 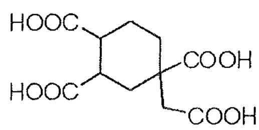

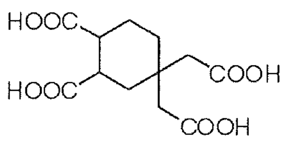
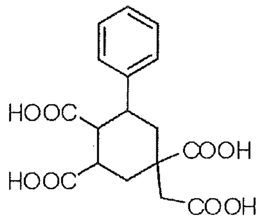
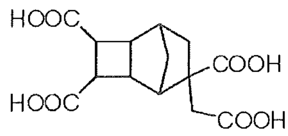

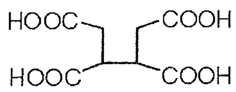
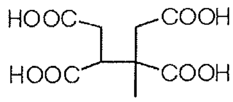
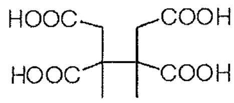

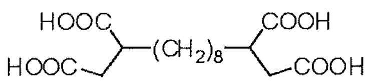
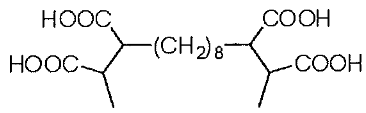

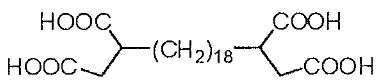
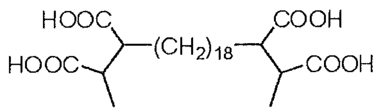

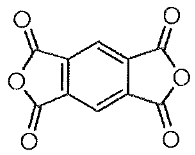
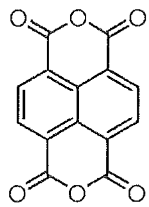
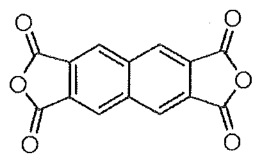

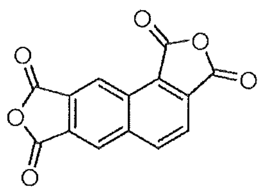
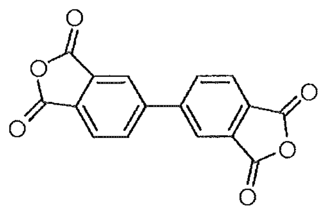
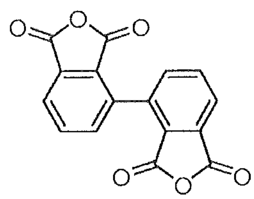

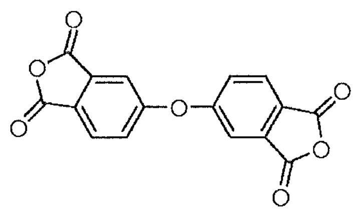
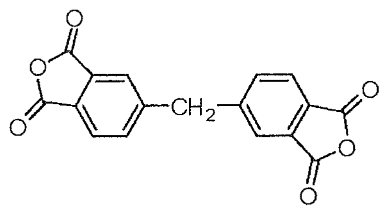

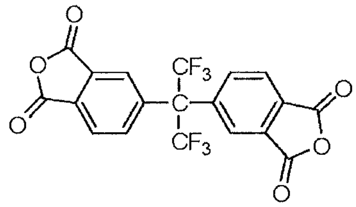
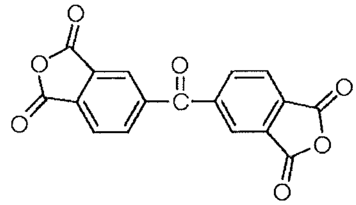

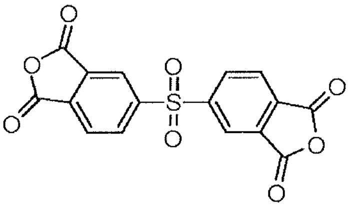

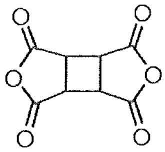
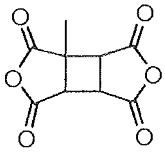
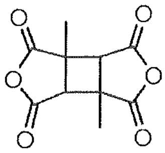

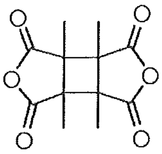
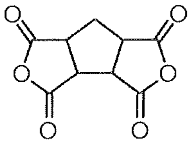
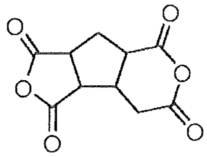

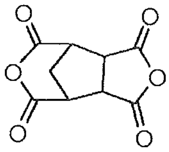
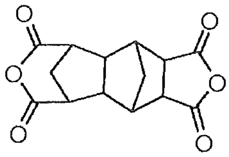

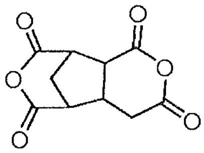
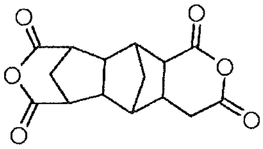

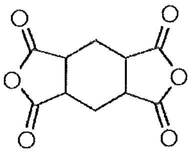
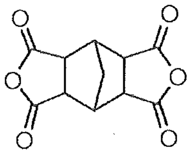
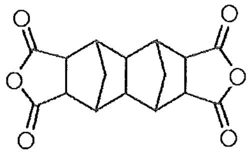

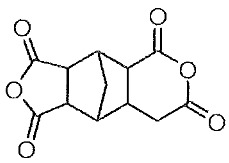
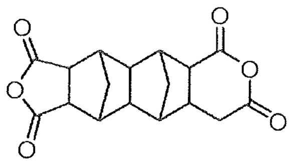

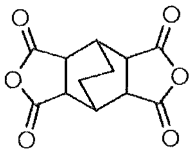
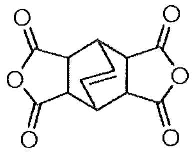
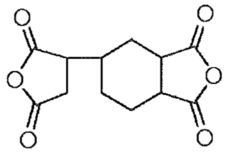

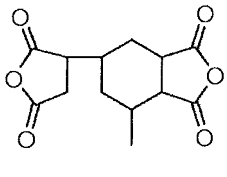
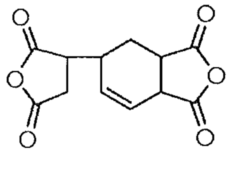
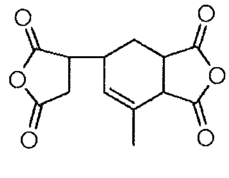

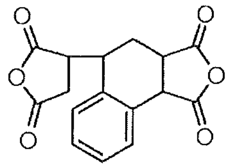
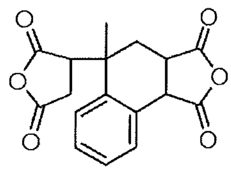

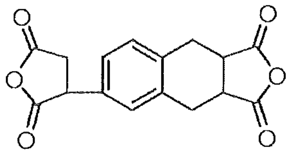
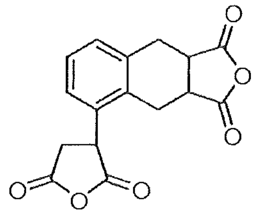
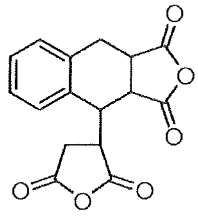

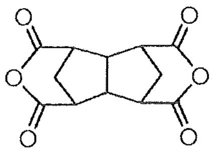
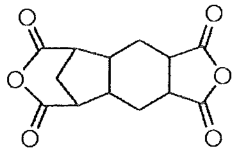
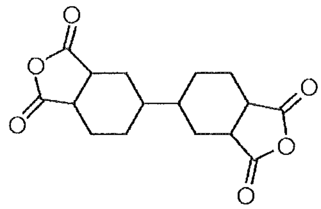

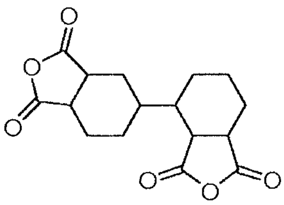
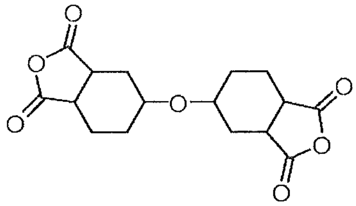

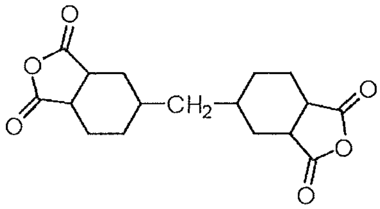
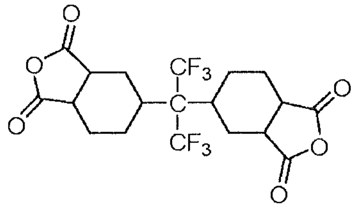

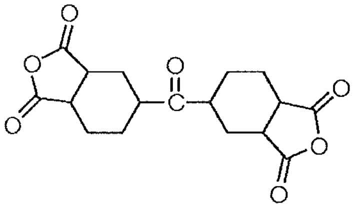
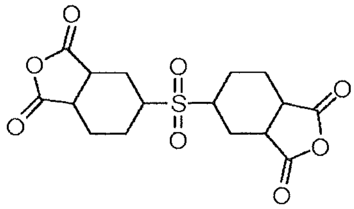

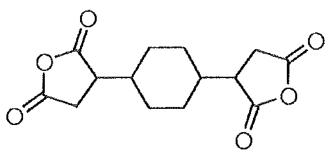
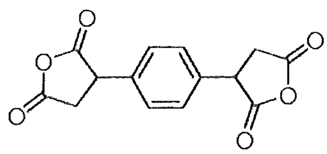

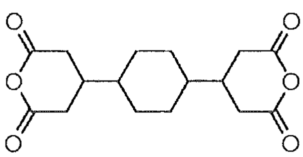
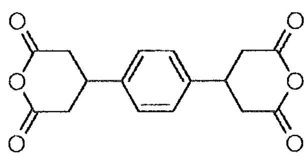

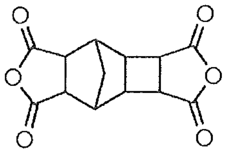
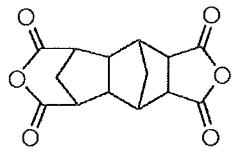
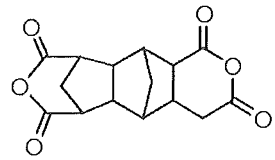

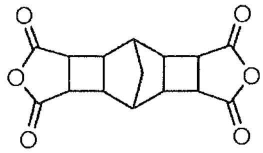
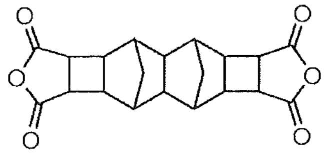

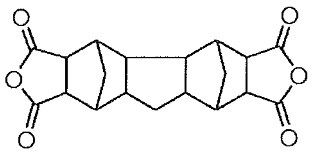

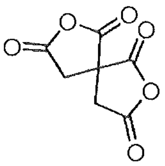
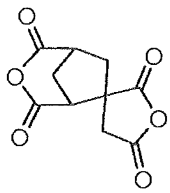
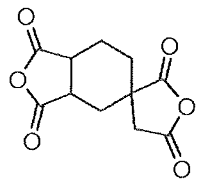

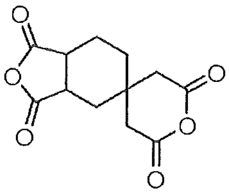
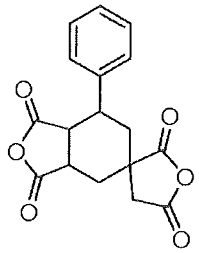
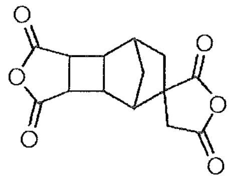

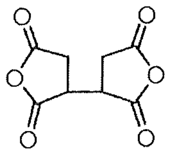
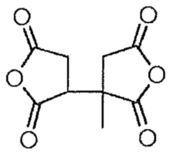
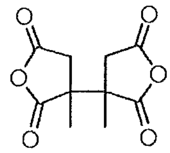

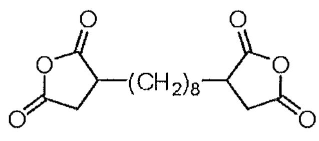
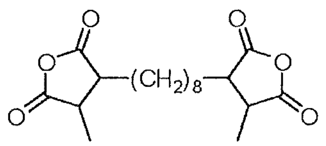

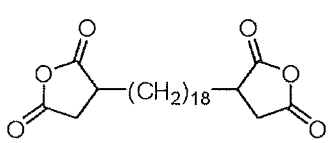
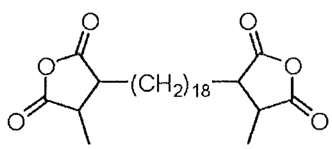

Among the tetracarboxylic acids or the tetracarboxylic acid anhydrides represented by the above chemical formulae, there are included, aromatic tetracarboxylic acids such as, for example, pyromellitic acid (1,2,4,5-benzene tetracarboxylic acid), 1,4,5,8-naphthalene tetracarboxylic acid, 2,3,6,7-naphthalene tetracarboxylic acid, 4,4'-oxydiphthalic acid, 1,1'-biphenyl-2,3,3',4'-tetracarboxylic acid, 3,3',4,4'-benzophenone tetracarboxylic acid and 3,3',4,4'-stilbene tetracarboxylic acid; aliphatic tetracarboxylic acids such as, for example, cyclobutane tetracarboxylic acid, methylcyclobutane tetracarboxylic acid, dimethylcyclobutane tetracarboxylic acid, tetramethylcyclobutane tetracarboxylic acid, cyclopentane tetracarboxylic acid, 1,2,4,5-cyclohexane tetracarboxylic acid, 2-carboxymethyl-1,3,4-cyclopentane tricarboxylic acid, 4-(1,2-dicarboxyethyl),-1,2,3,4-tetrahydronaphthalene-1,2-dicarboxylic acid and 5-(1,2-dicarboxyethyl)-3-methylcyclohexane-1,2-dicarboxylic acid; aromatic tetracarboxylic acid anhydrides such as, for example, pyromellitic anhydride (1,2,4,5-benzene tetracarboxylic acid anhydride), 1,4,5,8-naphthalene tetracarboxylic acid anhydride, 2,3,6,7-naphthalene tetracarboxylic acid anhydride, 4,4'-oxydiphthalic acid anhydride, 1,1'-biphenyl-2,3,3',4'-tetracarboxylic acid-2,3:3',4'-dianhydride, 3,3',4,4'-benzophenone tetracarboxylic acid anhydride and 3,3',4,4'-stilbene tetracarboxylic acid anhydride; aliphatic tetracarboxylic acid anhydrides such as, for example, cyclobutane tetracarboxylic acid anhydride, methylcyclobutane tetracarboxylic acid anhydride, dimethylcyclobutane tetracarboxylic acid anhydride, tetramethylcyclobutane tetracarboxylic acid anhydride, cyclopentane tetracarboxylic acid anhydride, 1,2,4,5-cyclohexane tetracarboxylic acid anhydride, 2-carboxymethyl-1,3,4-cyclopentane tricarboxylic acid-1,4:2,3-dianhydride, 4-(1,2-dicarboxyethyl),-1,2,3,4-tetrahydronaphthalene-1,2-dicarboxylic acid anhydride and 5-(1,2-dicarboxyethyl)-3-methylcyclohexane-1,2-dicarboxylic acid anhydride; and the like, as preferable tetracarboxylic acids or anhydrides of the tetracarboxylic acids. Among these preferable tetracarboxylic acids or anhydrides of the tetracarboxylic acids, because 2-carboxymethyl-1,3,4-cyclopentane tricarboxylic acid-1,4:2,3-dianhydride has high polymerization activity, it easily reacts with diamine derivatives represented by the above general formulae [1]-[3], and in addition, a polyamic acid or a polyimide prepared from a composition containing 2-carboxymethyl-1,3,4-cyclopentane tricarboxylic acid-1,4:2,3-dianhydride is included as the particularly preferable one in view of having high solubility to other organic solvents. It should be noted that specific examples represented by the above chemical formula names are only one of the examples, and any tetracarboxylic acids or tetracarboxylic acid anhydrides derived from the above formulae [4-A]-[4-i] can be used in the present invention, without being limited to specific examples exemplified here.

As the tetracarboxylic acid represented by the general formula [4] or the tetracarboxylic acid anhydride represented by the general formula [4'], it is preferable to use a tetracarboxylic acid anhydride represented by the general formula [4'], more specifically a tetracarboxylic acid dianhydride represented by the general formula [4'], in consideration of reactivity with the diamine derivatives represented by the general formulae [1]-[3].

As these tetracarboxylic acids or tetracarboxylic acid anhydrides, those synthesized appropriately by a common method may be used, or the commercially available one may also be used. For example, they may be synthesized by dehydrating under heating of tetracarboxylic acids in accordance with a method described in New Experimental Chemistry Course, first edition, Vol. 14, page 1123 to 1133, or the like. In addition, these tetracarboxylic acids or tetracarboxylic acid anhydrides may be used alone as one kind, or a plurality of kinds may be used in combination, as appropriate.

Use of these diamines represented by the general formulae [1]-[3] in a suitable ratio enables to express and maintain (control) pretilt angle at a suitable angle. As for this ratio, as the molar concentration where total molar number of the diamines represented by the general formulae [1]-[3] is set 100% by mol, content (molar concentration) of the meta-phenylene diamine derivative represented by the general formula [1] is usually 10 to 60% by mol, and preferably 20 to 50% by mol; content (molar concentration) of the meta-phenylene diamine derivative represented by the general formula [2] is usually 10 to 50% by mol, and preferably 10 to 30% by mol; and content (molar concentration) of the para-arylene diamine represented by the general formula [3] is usually 10 to 80% by mol, and preferably 30 to 70% by mol.

As described above, the composition for the liquid crystal alignment film of the present invention comprises the above components (A)-(D). Usually, the composition is provided in a form mixed with a suitable solvent. As the suitable solvent referred to herein, there are included, glycol-type solvents such as, for example, diethylene glycol and propylene glycol; ether-type solvents such as, for example, ethylene glycol monobutyl ether (butyl cellosolve); amide-type solvents such as, for example, formamide, acetamide and N-methylpyrrolidone (NMP); ester-type solvents such as, for example, γ-butyrolactone and γ-valerolactone; sulfoxide-type solvents such as, for example, dimethyl sulfoxide.

The polyamic acid or the polyimide of the present invention is the one obtained by reacting the components represented by the above (A)-(D).

The polyamic acid or the polyimide of the present invention can be produced, specifically, using a known method. For example, into a reactor equipped with raw materials inlet, nitrogen inlet, thermometer, stirrer and condenser, each of the diamines represented by the general formulae [1]-[3] is charged in a predetermined amount. Next, a suitable solvent such as, for example, N-methylpyrrolidone (NMP), γ-butyrolactone or the like, and a tetracarboxylic acid represented by the general formula [4] or a tetracarboxylic acid anhydride represented by the general formula [4'] are added. It should be noted that total charge amount of the tetracarboxylic acid or the tetracarboxylic acid anhydride is preferably nearly the same mole (about 0.9 to 1.1 in molar ratio) as total molar number of the diamines, and by adjusting the molar ratio, viscosity of the polyamic acid or the polyimide can also be controlled in a desired range. By reacting these solutions under stirring at a temperature of 50 to 80°C for 1 to 6 hours, a solution of the polyamic acid can be obtained.

It is desirable that the polyamic acid or the polyimide of the present invention has suitable viscosity. That is, it is desirable that viscosity of the polyamic acid or the polyimide is controlled in a range of 15 to 25 mPa•s, and preferably

in a range of 15 to 25 mPa•s. It should be noted that viscosity referred to here may be measured by a common method, and specifically, by using, for example, a rotating viscometer, for example, under the following measurement conditions. Instrument used: RE-80L (manufactured by Toki Sangyo Co.,Ltd), rotor code: 1, calibration standard solution: JS50, measurement temperature: 25°C, rotation number: 10 rpm, measurement time: 3 minutes, sample amount: 1.2 mL.

It is desirable that the polyamic acid is dehydration condensed to provide partial imidization, in using the polyamic acid of the present invention as use of a liquid crystal alignment film. It should be noted that the polyimide in the present invention can include two kinds: a complete polyimide, where the polyamic acid is completely imidized, and a partial polyimide where the polyamic acid is partially imidized. In the case of converting to the polyimide, it can be obtained by subjecting the obtained polyamic acid solution to an imidization reaction with an acid anhydride such as acetic anhydride, propionic anhydride trifluoro acetic anhydride or the like, which is a dehydrating agent, and a tertiary amine such as triethylamine, pyridine, collidine or the like, which is a dehydrating ring-closing catalyst, at a temperature of 60 to 120°C. Alternatively, a polyamic acid is precipitated from the obtained polyamic acid solution by using a large quantity of a poor solvent (alcohol-type solvents such as methanol, ethanol, isopropanol or the like, or glycol-type solvents), then the precipitated polyamic acid can be subjected to an imidization reaction in a solvent such as toluene, xylene or the like, together with the same dehydrating agent and dehydrating ring-closing catalyst described above at a temperature of 60 to 120 °C.

It is preferable that the polyimide (partial polyimide) of the present invention has suitable imidization rate, from the viewpoint that it is desirable to express and control pretilt angle, and still more to be provided with function of good electric characteristics or the like. Specific imidization rate is usually 1 to 90%, preferably 40 to 90%, and more preferably 60 to 90%. That is, by converting to a polyimide having such a preferable imidization rate, desirable function such as expression and maintaining (control) of optimum pretilt angle, or expression of effect to enhance electric characteristics can be expected.

It is preferable that ratio of a dehydration agent and a dehydration ring-closing catalyst, in the above imidization reaction, is 0.1 to 10 (molar ratio). It is preferable that total use amount of both is 1.5 to 10 times mole, relative to total molar amount of acids included in the tetracarboxylic acid or the tetracarboxylic acid anhydride to be used. Adjustment of the amount of the dehydration agent and the catalyst, reaction temperature and reaction time of this chemical imidization enables to control imidization degree, and thus the partial polyimide can be obtained. In the present invention, by the addition amount of acetic acid anhydride, imidization rate may be controlled. The obtained polyimide can be used with separating from a solvent to re-dissolve it into a solvent to be described later, or can be used without separating from a solvent. That is, the polyimide of the present invention includes both the one, where all acid amides of the polyamic acid are imidized, and the one, where acid amides of the polyamic acid are partially imidized (partial polyimide).

The obtained polyimide can be used by dilution with a solvent to adjust to the above desired viscosity. As the solvent to be used in this case, there are included, glycol-type solvents such as, for example, diethylene glycol and propylene glycol; ether-type solvents such as, for example, ethylene glycol monobutyl ether (butyl cellosolve); amide-type solvents such as, for example, formamide, acetamide and N-methylpyrrolidone (NMP); ester-type solvents such as, for example, γ-butyrolactone and γ-valerolactone; sulfoxide-type solvents such as, for example, dimethyl sulfoxide.

The polyamic acid may be identified by precipitating it using a large quantity of a poor solvent, completely separating a solid content and a solvent by filtration or the like, and analyzing it using IR or NMR. In addition, by boiling the obtained solid content in the poor solvent, weight average molecular weight can also be controlled in a desired range. Still more, a used monomer can be identified by decomposing the solid polyamic acid using an aqueous solution of strong alkali such as KOH and NaOH, extracting it using an organic solvent, and analyzing it using GC, HPLC or GC-MS.

The liquid crystal aligning agent of the present invention is the one including at least one or more kind of the above polyamic acid or a polyimide, and may include other components. As the other components, for example, a functional silane-containing compound and an epoxy-type cross-linking agent and the like are included. In addition, the liquid crystal alignment film not having a rib of the present invention from the liquid crystal aligning agent and the liquid crystal display element obtained by alignment division of the liquid crystal alignment film can be produced, for example, by the following method.

Firstly, by applying a solution including the liquid crystal aligning agent of the present invention, for example, by a printing method, to a transparent conductive film side of a substrate, where the transparent conductive film is provided, and heating it at a temperature of 80 to 200°C, preferably 120 to 200°C, a coated film is formed. This coated film is usually 0.001 to 1 μm, and preferably 0.005 to 0.5μ m.

As specific examples of a solvent to be mixed with the liquid crystal aligning agent (dissolve the liquid crystal aligning agent), in a solution including the above liquid crystal aligning agent, there are included, glycol-type solvents such as, for example, diethylene glycol and propylene glycol; ether-type solvents such as, for example, ethylene glycol monobutyl ether (butyl cellosolve); amide type solvents such as, for example, formamide, acetamide and N-methylpyrrolidone (NMP); ester type solvents such as, for example, γ-butyrolactone and γ-valerolactone; sulfoxide type solvents such as, for example, dimethyl sulfoxide.

The coated film formed as above is subjected to rubbing processing using a roll wound with cloth composed

of synthetic fiber such as Nylon. Next, a radiation sensitive resin composition is applied onto the liquid crystal alignment film by a spin coating method or a printing method, and heated at a temperature of 50 to 180°C, and preferably 50 to 120°C to form a coated film. This coated film is usually 0.1 to 10 μm, and preferably 0.3 to 5 μm.

Next, after irradiation of, for example, UV rays, onto the radiation sensitive resin composition on the obtained liquid crystal alignment film, via a mask having a predetermined pattern, development is performed using a developing solution to remove unnecessary parts, and resist patterns are formed to protect partially the liquid crystal alignment film.

Developing time is usually 10 to 240 seconds, and in addition, a developing method may be any of a liquid accumulation method, a dipping method, or the like. After the development, washing with flowing water is performed for 30 to 180 seconds, and air drying is performed using compressed air or compressed nitrogen.

Next, rubbing processing is performed on a substrate, which is formed with a resist pattern partially to protect the liquid crystal alignment film, in a reversed direction from the above. Then, the obtained substrate is washed with the above specific solvent to remove only the resist pattern.

Two substrate sheets formed with the liquid crystal alignment film in this way are arranged in an opposing way, so that a rubbing direction of the opposing parts of the liquid crystal alignment films become orthogonal or anti-parallel, a peripheral part between the substrates is sealed with a sealing agent, liquid crystal is filled, a filling hole is encapsulated, and then polarizing plates are adhered at both surfaces thereof, so that a polarizing direction coincides with a rubbing direction of the liquid crystal alignment film of each substrate or becomes orthogonal to a rubbing direction of the liquid crystal alignment film of each substrate, to obtain the liquid crystal display element.

As the above sealing agent, for example, a curing agent and an epoxy resin containing an aluminum oxide sphere, as a spacer, can be used.

In addition, as the above liquid crystal, Nematic type liquid crystals, Smectic type liquid crystals, or the like can be included, and among them, the one forming Nematic type liquid crystals are preferable and, for example, Schiff base-based liquid crystal, azoxy-based liquid crystal, biphenyl-based liquid crystal, phenylcyclohexane-based liquid crystal, ester-based liquid crystal, terphenyl-based liquid crystal, biphenylcyclohexane-based liquid crystal, pyrimidine-based liquid crystal, dioxane-based liquid crystal, bicyclooctane-based liquid crystal, cubane-based liquid crystal, or the like are used. In addition, these liquid crystals can be used by being added with a cholesteric liquid crystal such as, for example, cholestyl chloride, cholesteryl nonanoate, cholesteryl carbonate; or a chiral agent such as the commercially available one as a trade name of C-15, CB-15 (produced by Merck Ltd.) or the like. Further, ferroelectric liquid crystal such as, for example, p-decyloxybenzylidene-p-amino-2-methylbutyl cinnamate can also be used.

It should be noted that, as the polarizing plate to be used at the outer side of the liquid crystal display element, there can be included a polarizing plate which sandwiches a polarizing film called an H film, where iodine is absorbed while stretching and aligning polyvinyl alcohol, with a cellulose acetate protective film; a polarizing plate composed of the H film itself; or the like.

In this way, the liquid crystal alignment film and the liquid crystal display element of the present invention enable also to prepare simply and conveniently the liquid crystal alignment film and the liquid crystal display element, because of not requiring performing the step for laying a rib, such as present in a conventional method.

EXAMPLES

Explanation will be given below specifically on the present invention, based on Examples, however, the present invention should not be limited to these Examples. It should be noted that various measurements in Examples, Comparative Synthetic Examples and Comparative Examples were performed by the following methods.

- Measurement of imidization rate of a polyimide polymer -

The polyimide polymer was dissolved in deuterated dimethyl sulfoxide, to measure $^1$H-NMR (400MHz) at room temperature using tetramethylsilane as a standard substance, and it was calculated by the following expression (i).

$$\text{Expression (i)} \quad \text{Imidization rate (\%)} = \{1 - (A^1 / A^2) \times \alpha\} \times 100$$

$A^1$: Peak area derived from a proton of an NH group, in a precursor (polyamic acid) of a polymer (10 to 11 ppm)
$A^2$: Peak area derived from a proton derived from an aromatic ring (7 to 8 ppm) $\alpha$: Number of protons derived from an aromatic ring per one proton of the NH group, in the precursor (polyamic acid) of the polymer

- Measurement of solution viscosity of the polyimide polymer -

Solution viscosity (mPa•s) of the polyimide polymer was measured using an E-type rotation viscometer (manufactured by Toki Sangyo Co.,Ltd), as for a solution diluted so that a solid content concentration of the polyimide polymer becomes 7% by weight, using N-methylpyrrolidone (NMP) as a solvent, under condition of 25°C.

- Measurement of pretilt angle in the liquid crystal display element -

Pretilt angle was calculated by a crystal rotation method, using a pretilt angle measurement apparatus (PAS-301, manufactured by Toyo Corp.), under condition of inside a clean room (a temperature of 25°C, a humidity of 50%).

- Measurement of voltage retention rate in the liquid crystal display element -

After repeating application of a voltage of 5V twice onto the liquid crystal display element, under condition of an application time of 60 micro second and a span of 16.7 micro second, voltage retention rate after 16.7 millisecond from releasing the second application, was measured twice, using a liquid crystal property evaluation apparatus (6245 type, manufactured by Toyo Corp.), and average value thereof was calculated to adopt as the voltage retention rate.

Synthetic Example 1: Synthesis of 2,4-dinitrophenethyl acetate (2,4-DNEA)

Into 225 g of fuming nitric acid (3.24 mol; produced by Wako Pure Chemical Industries, Ltd.), 182 mL of 97% concentrated sulfuric acid (produced by Wako Pure Chemical Industries, Ltd.) was poured under stirring. This mixed acid was cooled with methanol / dry ice to maintain internal temperature at -15 to -5°C, and then 59.1 g of phenethyl acetate (PEA) (0.36 mol; produced by Toyotama Koryo Co., Ltd.) was dropped slowly into the mixed acid, while confirming the dispersion property of phenethyl acetate (PEA). After completion of the dropping, a refrigerant was changed to ice water to continue a reaction at 2 to 5°C for still more 4 hours. After confirming disappearance of phenethyl acetate (PEA) using thin layer chromatography (TLC), the reaction solution was poured in 740 g of ice water under stirring. After that, this solution was extracted with toluene (400 mL x 2), and then the organic layer was washed with 5% sodium bicarbonate water (400 mL) and dehydrated with anhydrous magnesium sulfate. Then, after this organic layer was filtered to remove magnesium sulfate, the filtrate was subjected to vacuum concentration followed by purification of the concentrated residue with silica gel column chromatography to obtain 85.5 g of yellow oil-like 2,4-dinitrophenethyl acetate (2,4-DNEA) (336 mol, yield: 93%). Measurement result using $^1$H-NMR is shown below.
$^1$H-NMR (400 MHz, $CDCl_3$) $\delta$ (ppm): 2.02 (3H, s), 3.39 (2H, t, J=6.4 Hz), 4.40 (2H, t, J=6.4 Hz), 7.64 (1 H, d, J=8.5 Hz), 8.41 (1 H, dd, J=8.5 Hz, 2.4 Hz), 8.77 (1 H, d, J=2.4 Hz).

Example 1: Synthesis of 2,4-diaminophenethyl acetate (2,4-DAEA)

After dissolving 21.16 g of 2,4-dinitrophenethyl acetate (2,4-DNEA) (83.2 mmol) into 100 mL of methanol, 1.06 g of 5% by weight palladium / carbon was added under nitrogen gas atmosphere to perform a reaction (reduction reaction) with hydrogen gas at room temperature for 24 hours under normal pressure condition. After confirming disappearance of 2,4-dinitrophenethyl acetate (2,4-DNEA) by TLC, palladium / carbon in the reaction solution was filtered out, and then this solution was subjected to vacuum concentration followed by purification of the concentrated residue with silica gel column chromatography. By adding ethyl acetate (13 mL) to 11.46 g of the obtained crude crystal, and heating it up to 55°C, the crude crystal was dissolved, and then n-hexane (35 mL) was poured slowly in this solution to precipitate a crystal. The precipitated crystal was filtered and the crystal was dried under reduced pressure to obtain 10.9 g of pale orange crystal of 2,4-diaminophenethyl acetate (2,4-DAEA) (56.1 mmol, yield: 68%). Measurement result using $^1$H-NMR is shown below.
$^1$H-NMR (400 MHz, $CDCl_3$) $\delta$ (ppm): 2.06 (3H, s), 2.73 (2H, t, J=7.6 Hz), 4.18 (2H, t, J=7.6 Hz), 6.04 (1 H, d, J=2.2 Hz), 6.08 (1H, dd, J=8.0 Hz, 2.2 Hz), 6.80 (1 H, d, J=8.0 Hz).

Synthetic Example 2: Synthesis of 2,4-dinitrophenethyl alcohol (2,4-DNPE)

After dissolving 25.68 g of crude 2,4-dinitrophenethyl acetate (2,4-DNEA) (121 mmol) obtained in accordance with a method of Synthetic Example 1 into 85 mL of methanol, 31. 5 mL of 36% hydrochloric acid was poured under stirring this solution. After pouring, internal temperature was held to 67°C for refluxing under heating for 4.5 hours. After completion of the reaction, the reaction solution was subjected to vacuum concentration, and 50 mL of ion exchanged water and 30 mL of a saturated sodium carbonate solution were dropped slowly onto the concentrated residue. After that, this solution was extracted with ethyl acetate (85 mL $\times$ 2), and then the organic layer was dehydrated with anhydrous

magnesium sulfate. Then, after this organic layer was filtered to remove magnesium sulfate, the filtrate was subjected to vacuum concentration, and then, after adding toluene (43 mL) to the concentrated residue (21.42 g), the solution was homogenized by heating to 45°C. After that, this solution was cooled to precipitate a crystal, and stirring was continued still more for 2 hours at -5°C. After stirring, the precipitated crystal was filtered and the crystal was dried under reduced pressure to obtain 15.41 g of pale orange crystal of 2,4-dinitrophenethyl alcohol (2,4-DNPE) (72.6 mmol, yield: 72%). Measurement result using $^1$H-NMR is shown below.
$^1$H-NMR (400 MHz, $CDCl_3$) $\delta$ (ppm): 1.79 (1H, t, J=5.2 Hz), 3.29 (2H, m), 4.00 (2H, q, J=5.6 Hz), 7.70 (1 H, d, J=8.4 Hz), 8.39 (1 H, dd, J=8.4 Hz, 2.4 Hz), 8.77 (1 H, d, J=2.4 Hz).

Synthetic Example 3: Synthesis of 2,4-dinitrophenethyl butyrate (2,4-DNEB)

After dissolving 100 g of 2,4-dinitrophenethyl alcohol (2,4-DNPE) (471 mmol) obtained in accordance with a method of Synthetic Example 2, and 52.46 g of triethylamine (518 mmol; produced by Wako Pure Chemical Industries, Ltd), into 100 mL of tetrahydrofuran (THF), 55.24 g of butyryl chloride (518 mmol; produced by Wako Pure Chemical Industries, Ltd) was dropped slowly taking 1 hour under ice cooling, so that internal temperature is below 15°C. After completion of the dropping, a reaction was continued under stirring at room temperature for 2 hours. After completion of the reaction, the reaction solution was extracted by pouring ethyl acetate (500 mL) and ion exchanged water (300 mL) thereto, and then the organic layer was washed with saturated sodium bicarbonate water (300 mL) and ion exchanged water (300 mL $\times$ 2), in this order, and after that, the organic layer after washing was subjected to vacuum concentration, followed by purification of the concentrated residue with silica gel column chromatography to obtain 78.84 g of yellow oil-like 2,4-dinitrophenethyl butyrate (2,4-DNEB) (279 mmol, yield: 59.3%). Measurement result using $^1$H-NMR is shown below.
$^1$H-NMR (400 MHz, $CDCl_3$) $\delta$ (ppm): 0.91 (3H, t, J=7.3 Hz), 1.60 (2H, m), 2.25 (2H, t, J=7.3 Hz), 3.40 (2H, t, J=6.4 Hz), 4.42 (2H, t, J=6.4 Hz), 7.67 (1 H, d, J=8.6 Hz), 8.42 (1 H, dd, J=8.6 Hz, 2.4 Hz), 8.80 (1 H, d, J=2.4 Hz).

Example 2: Synthesis of 2,4-diaminophenethyl butyrate (2,4-DAEB)

After dissolving 70.00 g (248 mmol) in 78.84 g of 2,4-dinitrophenethyl butyrate (2,4-DNEB) obtained in Synthetic Example 3 into 420 mL of methanol, 3.50 g of 5% by weight palladium / carbon was added under nitrogen gas atmosphere to perform a reaction (reduction reaction) with hydrogen gas at room temperature for 24 hours under normal pressure condition. After confirming disappearance of 2,4-dinitrophenethyl butyrate (2,4-DNEB) by TLC, palladium / carbon in the reaction solution was filtered out, and then this solution was subjected to vacuum concentration followed by purification of the concentrated residue with silica gel column chromatography to obtain 29.61 g of yellow oil-like 2,4-diaminophenethyl butyrate (2,4-DAEB) (133 mmol, yield: 54.3%). Measurement result using $^1$H-NMR is shown below.
$^1$H-NMR (400 MHz, $CDCl_3$) $\delta$ (ppm): 0.95 (3H, t, J=7.3 Hz), 1.65 (2H, m), 2.29 (2H, t, J=7.3 Hz), 2.73 (2H, t, J=6.4 Hz), 3.66 (4H, brs), 4.18 (2H, t, J=7.6 Hz), 6.03 (1 H, d, J=2.4 Hz), 6.07 (1 H, dd, J=8.1 Hz, 2.4 Hz), 6.80 (1 H, d, J=8.1 Hz).

Synthetic Example 4: Synthesis of 2,4-dinitrophenethyl methyl ether (2,4-DNPEM)

Into 55 mL of 97% concentrated sulfuric acid (produced by Wako Pure Chemical Industries, Ltd.), under ice cooling, 13.62 g of phenethyl methyl ether (100 mmol; produced by Toyotama Koryo Co., Ltd.) was slowly added, and after that, 16 g of fuming nitric acid (240 mmol; produced by Wako Pure Chemical Industries, Ltd.) was dropped taking 50 minutes, so that internal temperature is below 25°C. After completion of the dropping, disappearance of phenethyl methyl ether was confirmed by TLC, and then the reaction solution was poured slowly into ice water (250 mL) to extract this solution with ethyl acetate (100 mL). The obtained organic layer was washed with 10% saline (100 mL), 10% sodium bicarbonate water (100 mL), 10% saline (100 mL), in this order, and then dehydrated with anhydrous sodium sulfate. Next, this organic layer was filtered to remove sodium sulfate and then subjected to vacuum concentration, followed by purification of the concentrated residue with silica gel column chromatography to obtain 14.02 g of yellow oil-like 2,4-dinitrophenethyl methyl ether (2,4-DNPEM) (62.0 mmol, yield: 62%). Measurement result using $^1$H-NMR is shown below.
$^1$H-NMR (400 MHz, $CDCl_3$) $\delta$ (ppm): 3.29 (5H, m), 3.69 (2H, t, J=5.8 Hz), 7.66 (1H, d, J=8.5 Hz), 8.37 (1 H, dd, J=8.5 Hz, 2.4 Hz), 8.76 (1 H, d, J=2.4 Hz).

Synthetic Example 5: Synthesis of 2,4-diaminophenethyl methyl ether (2,4-DAPEM)

After dissolving 53.47 g of 2,4-dinitrophenethyl methyl ether (2,4-DNPEM) (236 mmol) obtained in accordance with a method of Synthetic Example 4, into 320 mL of methanol, 2.67 g of 5% by weight palladium / carbon was added under nitrogen gas atmosphere to perform a reaction (reduction reaction) with hydrogen gas at room temperature for 21 hours under normal pressure condition. After confirming disappearance of 2,4-dinitrophenethyl methyl ether (2,4-DNPEM) by TLC, palladium / carbon in the reaction solution was filtered out, and then this solution was subjected to vacuum concentration followed by purification of the concentrated residue with silica gel column chromatography to obtain 31.5 g of brown oil-like 2,4-diaminophenethyl methyl ether (2,4-DAPEM) (190 mmol, yield: 80%). Measurement result using $^1$H-NMR is shown below.
$^1$H-NMR (400 MHz, $CDCl_3$) δ (ppm): 2.69 (2H, t, J=6.4 Hz), 3.33 (3H, s), 3.56 (6H, brs, t, J=6.4 Hz), 6.04 (1 H, d, J=2.4 Hz), 6.09 (1 H, dd, J=8.0 Hz, 2.4 Hz), 6.80 (1 H, d, J=8.0 Hz).

Synthetic Example 6: Synthesis of β-cholestanyl 2,4-dinitrobenzoate

34.37 g of β-cholestanol (88.4 mmol, produced by Nikko Chemicals Co., Ltd), 21.00 g of 2,4-dinitrobenzoic acid (92.6 mmol, produced by Tokyo Chemical Industry Co., Ltd.) and 1.68 g of toluene sulfonic acid monohydrate (8.84 mmol, produced by Wako Pure Chemical Industries, Ltd.) were dissolved into 264 mL of toluene, and stirred for 3 hours under refluxing, and then it was confirmed that cholestanol became 1 % or lower using $^1$H-NMR. Next, after the reaction solution was cooled to room temperature, 400 mL of methanol was poured for crystallization at 3 to 5°C taking 1 hour. After that, the obtained precipitation was filtered and dried to obtain 46.48 g of β-cholestanyl 2,4-dinitrobenzoate (77.9 mmol, yield: 88%). Measurement result using $^1$H-NMR is shown below.
$^1$H-NMR (400 MHz, $CDCl_3$) δ (ppm): 0.64-1.94 (47H, m), 4.11 (2H, s), 4.73 (1H, m), 7.59 (1 H, d, J=8.0 Hz), 8.43 (1 H, d, J=8.0, 2.4 Hz), 8.94 (1 H, d, J=2.4 Hz).

Synthetic Example 7: Synthesis of β-cholestanyl 2,4-diaminobenzoate

30.00 g (50.3 mmol) in 46.48 g of β-cholestanyl 2,4-dinitrobenzoate obtained in Synthetic Example 6 was dissolved in 50 mL of tetrahydrofuran (THF), then inside of container was replaced with nitrogen, and 3.00 g of 5% palladium / carbon (produced by N.E. Chemcat Corp.) was added. After that, inside of container was replaced with hydrogen, to continue a reaction at room temperature for 40 hours under stirring. After confirming disappearance of the raw material by thin layer chromatography, palladium / carbon was filtered out. Next, this solution was subjected to vacuum concentration followed by purification with silica gel column chromatography (elution solvent: ethyl acetate / toluene = 1/2). The residue (22 g) was dissolved into 100 mL of dichloromethane, and washed with 100 mL of ion exchanged water five times. Further, the organic layer was subjected to vacuum concentration, and then the residue (21 g) was dissolved into 126 mL of THF, and 252 mL of methanol was poured therein. After crystallization by pouring 14 mL of ion exchanged water to continue stirring for 1 hour at room temperature, and still more1 hour at 3 to 5°C, the crystal was filtered and dried to obtain 18.91 g of β-cholestanyl 2,4-diaminobenzoate (35.2 mmol, yield: 70%). Measurement result using $^1$H-NMR is shown below.
$^1$H-NMR (400 MHz, $CDCl_3$) δ (ppm): 0.62-1.97 (47H, m), 3.40 (2H, s), 3.75 (4H, br, s), 4.66 (1 H, m), 6.06 (1 H, d, J=2.2 Hz), 6.10 (1 H, dd, J=7.8, 2.2 Hz), 6.85 (1 H, d, J=7.8 Hz).

Examples 3 to 8, and Comparative Synthetic Example 1: Synthesis of various kinds of the polyimide polymers

Diamine and tetracarboxylic acid dianhydride having a composition shown in Table 1, were added in this order into N-methylpyrrolidone (NMP), and then they were subjected to a reaction (polymerization) at 60°C for the period described in Table 1, so as to attain polymer concentration described in Table 1, to obtain an N-methylpyrrolidone (NMP) solution of a polyamic acid polymer. The obtained N-methylpyrrolidone (NMP) solution of the polyamic acid polymer was still more diluted by three times with 60 mL of N-methylpyrrolidone (NMP), and then acetic anhydride and pyridine were added in multiple mol number shown in Table 1, relative to the repeating unit of the polyamic acid polymer, to adjust the polymer concentration of the polyamic acid polymer to 10% by weight. This solution was heated to 110°C to be subjected to a reaction for 4 hours to perform dehydration ring-closing (imidization) of the polyamic acid polymer. After completion of the reaction, the solvent in the reaction container was replaced with new N-methylpyrrolidone (NMP) (by this handling, acetic anhydride and pyridine used in the reaction (imidization), were removed out of the container), to obtain N-methylpyrrolidone (NMP) solutions (PI-1 to PI-7) of the polyimide, having a solid content concentration of 30% by weight. Viscosity and imidization rate of these N-methylpyrrolidone (NMP) solutions (7% by weight) of the polyimide are shown in Table 1. It should be noted that as para-phenylene diamine, a commercial product of Wako Pure Chemical Industries, Ltd. was used, and as tetracarboxylic acid dianhydride (2-carboxymethyl-1,3,4-cyclopentane tricarboxylic acid-1,4:2,3-dianhydride), the one synthesized in accordance with a method described in JP-A-58-109479 was used.

[TABLE 1]

| | Polymer | Tetracarboxylic acid Dianhydride | | Diamine Compound | | Polyamic acid Polymer Concentration (wt%) | Polyamic acid Reaction Time (hr) | Acetic Anhydride (Times Mole) | Pyridine (Times Mole) | Imidization Rate (%) | Viscosity (mPa·s) |
|---|---|---|---|---|---|---|---|---|---|---|---|
| | | Abbreviation | Molar Fraction | Abbreviation | Molar Fraction | | | | | | |
| Example 3 | PI-1 | T-1 | 0.5 | D-1<br>D-2<br>D-3 | 0.1<br>0.2<br>0.2 | 30 | 6 | 2.4 | 2 | 75 | 19 |
| Example 4 | PI-2 | T-1 | 0.5 | D-1<br>D-2<br>D-3 | 0.1<br>0.3<br>0.1 | 30 | 1.5 | 2.4 | 5 | 68 | 21 |
| Example 5 | PI-3 | T-1 | 0.5 | D-1<br>D-2<br>D-4 | 0.1<br>0.2<br>0.2 | 30 | 6 | 4 | 2 | 85 | 14 |
| Example 6 | PI-4 | T-1 | 0.5 | D-1<br>D-2<br>D-4 | 0.10<br>0.25<br>0.15 | 30 | 4 | 3 | 2 | 79 | 15 |
| Example 7 | PI-5 | T-1 | 0.5 | D-1<br>D-2<br>D-4 | 0.1<br>0.3<br>0.1 | 30 | 3 | 2.8 | 2 | 75 | 17 |
| Example 8 | PI-6 | T-1 | 0.5 | D-1<br>D-2<br>D-5 | 0.1<br>0.2<br>0.2 | 30 | 1.5 | 1.8 | 2 | 69 | 16 |
| Comparative Synthetic Example 1 | PI-7 | T-1 | 0.5 | D-1<br>D-2 | 0.1<br>0.4 | 20 | 1.5 | 1.4 | 2 | 60 | 20 |

-Tetracarboxylic acid Dianhydride-

T-1: 2-Carboxymethyl-1,3,4-cyclopentane tricarboxylic acid-1,4;2,3 dianhydride

-Diamine Compound-

D-1: β-Cholestanyl 3,5-Diaminobenzoate

D-2: para-Phenylene diamine

D-3: 2,4-Diaminophenethyl acetate

D-4: 2,4-Diaminophenethyl butyrate

(continued)

| | Polymer | Tetracarboxylic acid Dianhydride | | Diamine Compound | | Polyamic acid Polymer Concentration (wt%) | Polyamic acid Reaction Time (hr) | Acetic Anhydride (Times Mole) | Pyridine (Times Mole) | Imidization Rate (%) | Viscosity (mPa·s) |
|---|---|---|---|---|---|---|---|---|---|---|---|
| | | Abbreviation | Molar Fraction | Abbreviation | Molar Fraction | | | | | | |
| D-5: 2,4-Diaminophenethyl methyl ether | | | | | | | | | | | |

Example 9: Preparation of the liquid crystal alignment film and the liquid crystal display element of the present invention

After adding N,N,N',N'-tetraglycidyl-4,4'-diaminodiphenylmethane (MY-721) into an N-methylpyrrolidone (NMP) solution (PI-1) of the polyimide obtained in Example 3, so as to attain 10 parts by weight relative to 100 parts by weight of the total polymer, it was dissolved in the mixed solvent of N-methylpyrrolidone (NMP) / butyl cellosolve (ethylene glycol monobutyl ether) (BC), to prepare a solution having a solvent composition of NMP / BC = 50 / 50 in weight ratio, and a solid content concentration of 4% by weight. Next, by filtration using a membrane filter having a pore size of 0.5 μm, a liquid crystal alignment film material was obtained. The obtained liquid crystal alignment film material was applied using a spinner, onto a transparent conductive film composed of an ITO film, provided at one surface of a glass substrate having a thickness of 1 mm, and baked at 70°C for 2 minutes and further at 220°C for 20 minutes to form a coated film having a film thickness of 70 nm. Then, by performing rubbing processing using a rubbing machine (small-size rubbing device), manufactured by E.H.C. Co., Ltd., having a roll which was wound with rayon cloth, liquid crystal aligning capability was given to the coated film, and the liquid crystal alignment film of the present invention was obtained. It should be noted that the rubbing processing was performed under the following condition: Roller rotation number: 300 rpm, stage transfer speed: 600 mm / min, and pile's indentation length: 0.1 mm. Two substrate sheets formed with the liquid crystal alignment film of the present invention as above were prepared, and at the outer peripheral part of each substrate, epoxy resin-type adhesives (STRUCT BOND, produced by Mitsui Chemicals, Inc.) containing a gap agent was applied a 10 μm, and then the two substrate sheets were arranged in an opposing way via a gap, and the outer peripheral parts themselves were abutted and clamped to harden the adhesives. Next, after filling a negative type liquid crystal (ZLI-4792, produced by Merck Ltd.) from a liquid crystal inlet provided in advance, into the cell gap between the substrates for the above liquid crystal display element, by utilizing capillary phenomenon, and by sealing the inlet and exit with photo-curing adhesives, the liquid crystal display element was prepared. By evaluation result of the obtained liquid crystal display element, it has been confirmed to be the good one, having pretilt angle of 84 degree and a voltage retention rate of 99%.

Examples 10 to 14, and Comparative Example 1: Preparation of the liquid crystal alignment film and the liquid crystal display element

Similarly as in Example 9, a solution having a solid content concentration of 4% by weight was prepared from the N-methylpyrrolidone (NMP) solutions (PI-2 to PI-7) of the polyimide polymer obtained in Examples 4 to 8 and Comparative Synthetic Example 1, to prepare the liquid crystal alignment film as well as the liquid crystal display element. As for the obtained liquid crystal display element, pretilt angle and voltage retention rate were measured, respectively. The results thereof are shown in Table 2, together with the result of Example 9.

[TABLE 2]

| Example | Polymer | Pretilt angle (°) | Voltage Retention Rate (%) |
|---|---|---|---|
| Example 9 | PI-1 | 84 | 99 |
| Example 10 | PI-2 | 85 | 99 |
| Example 11 | PI-3 | 77 | 99 |
| Example 12 | PI-4 | 82 | 99 |
| Example 13 | PI-5 | 86 | 99 |
| Example 14 | PI-6 | 86 | 99 |
| Comparative Example 1 | PI-7 | 89 | 99 |

As is clear from the above results, it has been clarified that in the liquid crystal display element provided with the liquid crystal alignment film prepared from the polyimide polymer obtained from the composition for forming the liquid crystal alignment film of the present invention, a liquid crystal molecule contained in the element is inclined a little less than 90 degree (77 to 86 degree), along a rubbing direction of the liquid crystal alignment film. On the other hand, it has been clarified that in the liquid crystal display element provided with the liquid crystal alignment film prepared from the polyimide polymer obtained from the composition not including meta-phenylene diamine represented by the general formula [1], a liquid crystal molecule contained in the element is little inclined. The liquid crystal display element of the present invention, where the liquid crystal molecule is inclined in 77 to 86 degree relative to a substrate, shows similar behavior (alignment) as in the liquid crystal display element containing the liquid crystal molecule in no application of voltage, as if in the rib-slit method. That is, pretilt angle required to an alignment film in the TN (Twisted Nematic) mode

is about 3 to 6 degree (for example, as described in J. Photopolym. Sci. Technol., Vol. 24, No. 3, page 2011.), and pretilt angle required in the case where this is aligned vertically is about 84 to 87 degree. Accordingly, in application of the liquid crystal display element showing such behavior (alignment) to the MVA mode, by adjusting appropriately a rubbing direction of the liquid crystal alignment film inside one pixel or inside one sub-pixel, alignment division can be attained, and in addition, a liquid crystal display device of a mode showing the same behavior as if in the rib-slit method can be provided, by application / no application of voltage, because the liquid crystal molecule can be inclined in a horizontal direction relative to a substrate in application of voltage. In addition, unlike the rib-slit method, because of no scattering of light by the rib, a liquid crystal display device can be provided, which does not raise a problem of deteriorating light utilization efficiency (aperture ratio becomes lower), or decreasing contrast ratio caused by leakage of backlight from the slit.

In addition, in the liquid crystal display element provided with the liquid crystal alignment film prepared from the polyimide polymer obtained from the composition for forming the liquid crystal alignment film of the present invention, such effect can be expected that a display is high quality and has low impurity, and has superior display performance, because of having a very high voltage retention rate thereof of 99%.

INDUSTRIAL APPLICABILITY

The composition for the liquid crystal alignment film of the present invention is the one to be used suitably as the liquid crystal alignment film material, which enables to control pretilt angle of a liquid crystal molecule in no application of voltage. In addition, the liquid crystal alignment film prepared from the liquid crystal aligning agent comprising the polyamic acid or the polyimide obtained by reacting such the composition enables to hold the alignment of a liquid crystal molecule in an inclined state in several degrees from a vertical direction to a horizontal direction relative to a substrate, depending on a rubbing direction of the liquid crystal alignment film in no application of voltage. Accordingly, the liquid crystal display element provided with such the liquid crystal alignment film is the one which can be used suitably as a liquid crystal display device of such the MVA mode.

Still more, the method for producing the liquid crystal alignment film of the present invention is a suitable method as a method for producing the liquid crystal alignment film for a vertical alignment-type liquid crystal display element, which enables to regulate an alignment of a liquid crystal molecule, as well as control pretilt angle of the liquid crystal molecule within a range of 75 to 87 degree.

## Claims

**1.** A composition for a liquid crystal alignment film comprising components represented by the following (A)-(D):

(A) a meta-phenylene diamine derivative represented by the general formula [1];

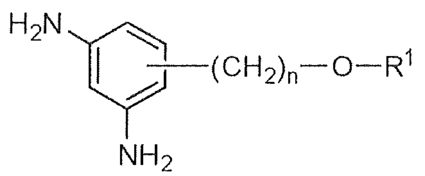

[1]

(wherein $R^1$ represents an alkyl group having 1 to 6 carbon atoms or an acyl group having 2 to 7 carbon atoms, and n represents an integer of 1 to 3.),

(B) a meta-phenylene diamine derivative represented by the general formula [2];

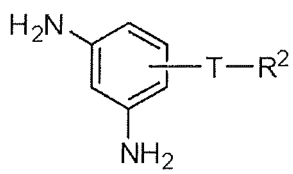

[2]

(wherein $R^2$ represents an alkyl group having 8 to 20 carbon atoms or a group having a steroid skeleton, and T represents an oxygen atom or a carbonyloxy group.),

(C) a para-arylene diamine represented by the general formula [3];

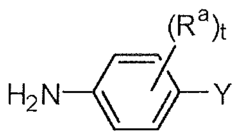

[3]

(wherein Y represents an amino group, an 4-aminophenyl group or an 4-aminophenylmethyl group, t moieties of $R^a$ represent each independently an alkyl group having 1 to 3 carbon atoms, an aryl group having 6 to 10 carbon atoms or an aralkyl group having 7 to 12 carbon atoms, and t represents an integer of 0 to 4. It should be noted that a part or all of t moieties of $R^a$ may be a substituent of a phenyl group in an 4-aminophenyl group or an 4-aminophenylmethyl group represented by Y.),
(D) a tetracarboxylic acid represented by the general formula [4] or a tetracarboxylic acid anhydride represented by the general formula [4'];

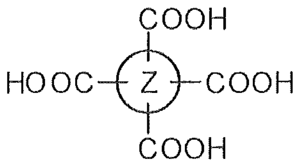

[4]

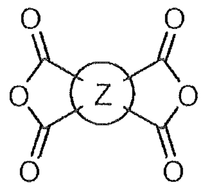

[4']

(wherein Z represents a tetravalent hydrocarbon group.).

2. The composition according to claim 1, wherein molar concentration of the (A), (B) and (C) is 10 to 60% by mole, 10 to 50% by mole and 10 to 80% by mole, respectively.

3. The composition according to claim 1, wherein the (A) is the one represented by the general formula [1']:

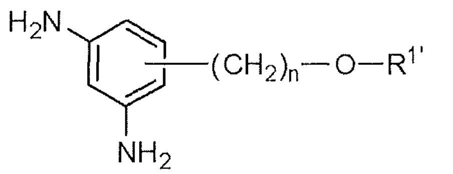

[1']

(wherein $R^{1'}$ represents an acyl group having 2 to 7 carbon atoms, and n represents an integer of 1 to 3.).

4. The composition according to claim 1, wherein the (A) is the one represented by the general formula [1'-A]:

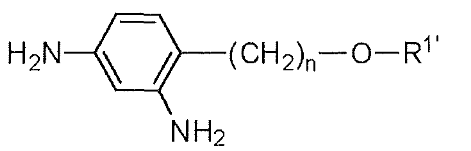

[1'-A]

(wherein $R^{1'}$ represents an acyl group having 2 to 7 carbon atoms, and n represents an integer of 1 to 3.).

5. The composition according to claim 1, wherein the (A) is 2,4-diaminophenethyl acetate or 2,4-diaminophenethyl butyrate.

6. The composition according to claim 1, wherein the (B) is the one represented by the general formula [2']:

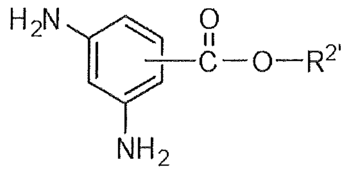

[2']

(wherein $R^{2'}$ represents a group having a steroid skeleton.).

**7.** The composition according to claim 1, wherein the (B) is β-cholestanyl 2,4-diaminobenzoate, β-cholestanyl 3,5-diaminobenzoate or β-cholestanyl 2,4-diaminophenol.

**8.** The composition according to claim 1, wherein the (B) is β-cholestanyl 2,4-diaminobenzoate or β-cholestanyl 3,5-diaminobenzoate.

**9.** The composition according to claim 1, wherein the (C) is para-phenylene diamine.

**10.** The composition according to claim 1, wherein the (D) is 2-carboxymethyl-1,3,4-cyclopentane tricarboxylic acid-1,4:2,3-dianhydride.

**11.** The composition according to claim 1, wherein the composition is the one to be used for forming a liquid crystal alignment film for a vertical alignment-type liquid crystal display element.

**12.** A polyamic acid or a polyimide obtained by reacting the components represented by the following (A)-(D):

(A) a meta-phenylene diamine derivative represented by the general formula [1];

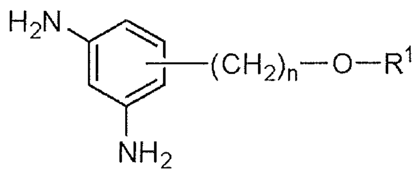

[1]

(wherein $R^1$ represents an alkyl group having 1 to 6 carbon atoms or an acyl group having 2 to 7 carbon atoms, and n represents an integer of 1 to 3.),
(B) a meta-phenylene diamine derivative represented by the general formula [2];

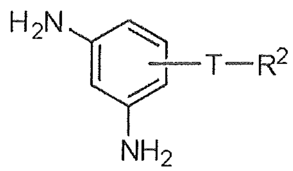

[2]

(wherein $R^2$ represents an alkyl group having 8 to 20 carbon atoms or a group having a steroid skeleton, and T represents an oxygen atom or a carbonyloxy group.),
(C) a para-arylene diamine represented by the general formula [3];

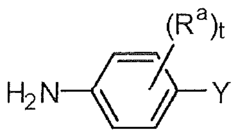

[3]

(wherein Y represents an amino group, an 4-aminophenyl group or an 4-aminophenylmethyl group, t moieties of $R^a$ represent each independently an alkyl group having 1 to 3 carbon atoms, an aryl group having 6 to 10 carbon atoms or an aralkyl group having 7 to 12 carbon atoms, and t represents an integer of 0 to 4. It should

be noted that a part or all of t moieties of $R^a$ may be a substituent of a phenyl group in an 4-aminophenyl group or an 4-aminophenylmethyl group represented by Y.),
(D) a tetracarboxylic acid represented by the general formula [4] or a
tetracarboxylic acid anhydride represented by the general formula [4'];

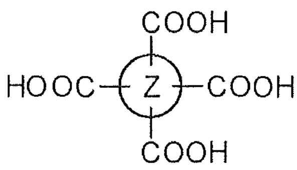 [4] 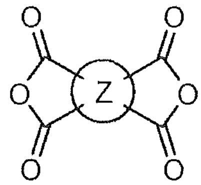 [4']

(wherein Z represents a tetravalent hydrocarbon group.).

**13.** The polyamic acid or the polyimide according to claim 12, wherein molar concentration of the (A), (B) and (C) is 10 to 60% by mole, 10 to 50% by mole and 10 to 80% by mole, respectively.

**14.** A liquid crystal aligning agent comprising the polyamic acid or the polyimide according to claim 12.

**15.** A liquid crystal alignment film prepared from the liquid crystal aligning agent according to claim 14.

**16.** A liquid crystal display element, **characterized by** being provided with the liquid crystal alignment film according to claim 15.

**17.** A compound represented by the general formula [1']:

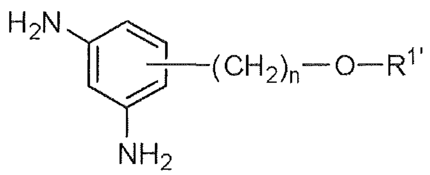 [1']

(wherein $R^{1'}$ represents an acyl group having 2 to 7 carbon atoms, and n represents an integer of 1 to 3.).

**18.** The compound according to claim 17, wherein the compound is the one to be used for controlling pretilt angle of a liquid crystal molecule, in no application of voltage, on a liquid crystal alignment film at 75 to 87 degree.

**19.** A method for producing a liquid crystal alignment film for a vertical alignment-type liquid crystal display element, comprising a step for forming a coated film by applying a solution of a polyamic acid or a polyimide obtained by reacting the components represented by the following (A)-(D) onto a support, and a step for performing rubbing processing of the surface of the coated film:

(A) a meta-phenylene diamine derivative represented by the general formula [1];

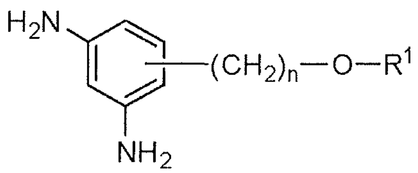 [1]

(wherein $R^1$ represents an alkyl group having 1 to 6 carbon atoms or an acyl group having 2 to 7 carbon atoms, and n represents an integer of 1 to 3.),
(B) a meta-phenylene diamine derivative represented by the general formula [2];

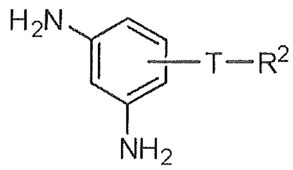

[2]

(wherein $R^2$ represents an alkyl group having 8 to 20 carbon atoms or a group having a steroid skeleton, and T represents an oxygen atom or a carbonyloxy group.),
(C) a para-arylene diamine represented by the general formula [3];

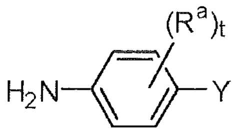

[3]

(wherein Y represents an amino group, an 4-aminophenyl group or an 4-aminophenylmethyl group, t moieties of $R^a$ represent each independently an alkyl group having 1 to 3 carbon atoms, an aryl group having 6 to 10 carbon atoms or an aralkyl group having 7 to 12 carbon atoms, and t represents an integer of 0 to 4. It should be noted that a part or all of t moieties of $R^a$ may be a substituent of a phenyl group in an 4-aminophenyl group or an 4-aminophenylmethyl group represented by Y.),
(D) a tetracarboxylic acid represented by the general formula [4] or a tetracarboxylic acid anhydride represented by the general formula [4'];

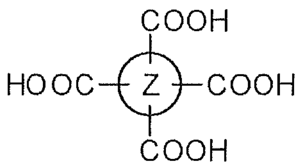

[4]

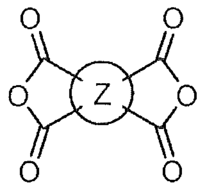

[4']

(wherein Z represents a tetravalent hydrocarbon group.).

**20.** The method for producing according to claim 19, wherein the step for forming the coated film is a step for forming a coated film by applying a solution of a polyimide.

**21.** The method for producing according to claim 19, wherein a liquid crystal alignment film is obtained which enables to control pretilt angle of a liquid crystal molecule, in no application of voltage, at 75 to 87 degree.

**INTERNATIONAL SEARCH REPORT**

International application No.
PCT/JP2013/057039

A. CLASSIFICATION OF SUBJECT MATTER
*G02F1/1337*(2006.01)i, *C08G73/10*(2006.01)i

According to International Patent Classification (IPC) or to both national classification and IPC

B. FIELDS SEARCHED

Minimum documentation searched (classification system followed by classification symbols)
G02F1/1337, C08G73/10

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2013 |
| Kokai Jitsuyo Shinan Koho | 1971-2013 | Toroku Jitsuyo Shinan Koho | 1994-2013 |

Electronic data base consulted during the international search (name of data base and, where practicable, search terms used)
CAplus(STN), REGISTRY(STN)

C. DOCUMENTS CONSIDERED TO BE RELEVANT

| Category* | Citation of document, with indication, where appropriate, of the relevant passages | Relevant to claim No. |
|---|---|---|
| X<br>A | WO 2009/154208 A1 (Nissan Chemical Industries, Ltd.),<br>23 December 2009 (23.12.2009),<br>examples 6, 7, 13, 14, 17 to 20, 23, 24<br>& CN 102067024 A & TW 201005007 A<br>& KR 10-2011-0017908 A | 17,18<br>1-16,19-21 |
| X<br>A | WO 2011/74546 A1 (Nissan Chemical Industries, Ltd.),<br>23 June 2011 (23.06.2011),<br>examples 5, 6<br>& CN 102754020 A & TW 201132745 A<br>& KR 10-2012-0103629 A | 17,18<br>1-16,19-21 |

☐ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 09 April, 2013 (09.04.13) | 16 April, 2013 (16.04.13) |

| Name and mailing address of the ISA/ | Authorized officer |
|---|---|
| Japanese Patent Office | |
| Facsimile No. | Telephone No. |

Form PCT/ISA/210 (second sheet) (July 2009)

**INTERNATIONAL SEARCH REPORT**

International application No.
PCT/JP2013/057039

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:

The only technical feature common to the invention of claim 1 and the respective inventions of claims 17 and 18 is "a methaphenylene diamine derivative represented by general formula [1] in claim 1".

The above-said technical feature cannot be considered to be a special technical feature, since the technical feature does not make a contribution over the prior art in the light of the contents disclosed in WO 2009/154208 A1 and WO 2011/74546 A1.

Further, there is no other same or corresponding special technical feature between these inventions.

(Continued to extra sheet)

1. ☐ As all required additional search fees were timely paid by the applicant, this international search report covers all searchable claims.

2. ☒ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☐ No required additional search fees were timely paid by the applicant. Consequently, this international search report is restricted to the invention first mentioned in the claims; it is covered by claims Nos.:

**Remark on Protest**

☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (July 2009)

**INTERNATIONAL SEARCH REPORT**

International application No.
PCT/JP2013/057039

Continuation of Box No.III of continuation of first sheet(2)

Accordingly, the following two inventions are involved in claims.
(Invention 1) the inventions of claims 1-16 and 19-21
(Invention 2) the inventions of claims 17 and 18

Form PCT/ISA/210 (extra sheet) (July 2009)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 11242225 A
- JP 2005316363 A
- JP 58109479 A


### Non-patent literature cited in the description


- *Illustrated introduction, Easy understandable basics and mechanism of the newest display technology,* ISBN 978-4-7980-2375-5
- New Experimental Chemistry Course. vol. 14, 1123-1133
- *J. Photopolym. Sci. Technol.,* vol. 24 (3), 2011